# EUROPEAN PATENT SPECIFICATION

(11) **EP 1 969 367 B1**
(45) Date of publication and mention of the grant of the patent: **10.08.2011**
(21) Application number: 06848996.2
(22) Date of filing: 12.12.2006
(51) Int. Cl.: G01N 33/50, C12N 15/10

(54) **ASSAY FOR PARKINSON'S DISEASE THERAPEUTICS**
TEST FÜR MORBUS-PARKINSON-THERAPEUTIKA
ANALYSE VISANT À IDENTIFIER DES TRAITEMENTS DE LA MALADIE DE PARKINSON

(30) Priority: 12.12.2005 US 749964 P
(43) Date of publication of application: 17.09.2008
(73) Proprietor: Elan Pharmaceuticals Inc., South San Francisco, CA 94080 (US)
(72) Inventor: JOHNSTON, Jennifer, A., Mill Valley, California 94941 (US)
(74) Representative: Mercer, Christopher Paul
(86) International application number: PCT/US2006/047515
(87) International publication number: WO 2007/089334

(56) References cited:
- EP-A1- 1 416 272
- WO-A-02/079459
- US-A1- 2004 214 763
- US-A1- 2005 042 607
- BANDOPADHYAY ET AL: "Synphilin-1 and parkin show overlapping expression patterns in human brain and form aggresomes in response to proteasomal inhibition" NEUROBIOLOGY OF DISEASE, BLACKWELL SCIENCE, OXFORD, GB, vol. 20, no. 2, November 2005 (2005-11), pages 401-411, XP005118674 ISSN: 0969-9961
- TANAKA KEIJI ET AL: "Parkin is linked to the ubiquitin pathway" JOURNAL OF MOLECULAR MEDICINE (BERLIN), vol. 79, no. 9, September 2001 (2001-09), pages 482-494, XP002440053 ISSN: 0946-2716
- MATSUDA NORIYUKI ET AL: "Diverse effects of pathogenic mutations of parkin that catalyze multiple monoubiquitylation in vitro" JOURNAL OF BIOLOGICAL CHEMISTRY, vol. 281, no. 6, 8 December 2005 (2005-12-08), pages 3204-3209, XP002453916 ISSN: 0021-9258

## Description

### FIELD OF THE INVENTION

The invention relates to assays for agents useful for treatment of Parkinson's Disease . The invention finds application in the fields of protein purification, drug discovery, and medicine.

### BACKGROUND

Parkinson's disease (PD) is a neurological disorder characterized neuropathologically as a loss of dopamine neurons of the substantia nigra. This neuronal loss manifests clinically as alterations in movement, such as Bradykinesia, rigidity and/or tremor (Gelb et al., 1999, Arch. Neurol. 56: 33-39). Analysis of human genetic data has been used to characterize genes linked to the development of PD. One of these genes was localized to chromosome 6 using a cohort of juvenile onset patients and identified specifically as Parkin protein (Kitada et al., 1998, Nature 392: 605-608). Parkin protein has been shown to be an E3 ligase protein that functions in the ubiquitin-proteasome system (UPS) (Shimura, 2000, Nature Genetics 25:302-305). The UPS is a major cellular pathway involved in the targeted removal of proteins for degradation and E3 ligases function to identify and label substrates for degradation by cellular proteasomes (Hereshko and Cienchanover, 1998, Ann. Rev. Biochem. 67;425-479) or . lysosomes (Hicke, 1999, Trends in Cell Biology 9:107-112).

There is an urgent need for new methods for treating Parkinson's disease. The present invention provides methods that are useful for identifying and/or validating agents for PD therapy.

### BRIEF SUMMARY OF THE INVENTION

In one aspect, the invention provides assays for identification of, or screening for, compounds useful for treatment of Parkinson's Disease (PD). The invention provides a cell-based assay for identifying a candidate compound for treatment of Parkinson's Disease including (a) exposing a mammalian cell expressing Parkin to a test agent; and (b) comparing proteasome function in the cell with proteasome function characteristic of a corresponding mammalian cell expressing Parkin not exposed to the test agent, where an increased level of proteasome function in the cell exposed to the test agent indicates the agent is a candidate compound for treatment of Parkinson's Disease.

Various methods may be used to measure or assess proteasome function. In one embodiment, the mammalian cells express GFPu and proteasome function is measured by measuring the amount of GFPu in the cells. In one embodiment the amount of GFPu in the cells is determined by measuring GFPu fluorescence.

In some cases the cell-based screening method also includes a proteasome function assay including (i) exposing a mammalian cell expressing a mutant Parkin to the candidate compound; and (ii) comparing proteasome function in the cell in with proteasome function characteristic of a cell expressing a mutant Parkin not exposed to the candidate compound.

In some cases the cell-based screening method also includes a proteasome function assay including (i) exposing a mammalian cell expressing another protein, such as Huntingtin, to the candidate compound; and (ii) comparing proteasome function in the cell with proteasome function characteristic of a cell expressing the other protein and not exposed to the candidate compound.

In some cases the cell-based screening method also includes an *in vitro* activity assay including (i) measuring the autoubiquitination activity of a purified Parkin protein in the presence of the compound; and (ii) comparing the autoubiquitination activity of purified Parkin protein in the presence of the compound with autoubiquitination activity of purified Parkin protein in the absence of the compound.

In some cases the cell-based screening method also includes an in vitro activity binding assay including (i) contacting the compound with purified Parkin protein and (ii) detecting the binding, if any, of the compound and the Parkin protein.

### BRIEF DESCRIPTION OF THE FIGURES

Figure 1 shows an immunoblot demonstrating that overexpression of Parkin results in impaired proteasome activity.

Figure 2A-E shows epifluorescent and immunofluorscent images illustrating that expression of Parkin protein leads to stabilization and aggregation of other proteasome substrates such as GFPu.

Figure 3 shows FACscan analysis of GFPu levels in cells expressing GFPu and transfected with a vector expression Parkin or a Parkin mutant (2ug DNA). The bar graph shows GFPu levels 2 days post transfection. Mutants 167, 212, 275 and 289 decreased proteasome activity above the wild-type Parkin (PKN).

Figure 4 shows formation of GFPu aggresomes after expression of various Parkin mutant cDNAs in HEK293/GFPu cells. Cells were transfected with 2ug cDNA and five days later epifluorescence images of each sample were recorded using the same camera settings for each sample to reflect the level of fluorescence intensity. Fluorescence intensity is a direct measure of GFPu levels in the cells.

Figure 5A-B shows the distribution of Parkin protein in normal and PD brains.

Figure 6 shows fluorescent images of cells transfected with a vector control (Fig. 6A, left) or wild type Parkin or (Fig. 6A, right) and the average fluorescence intensities from the cells (Fig. 6B).

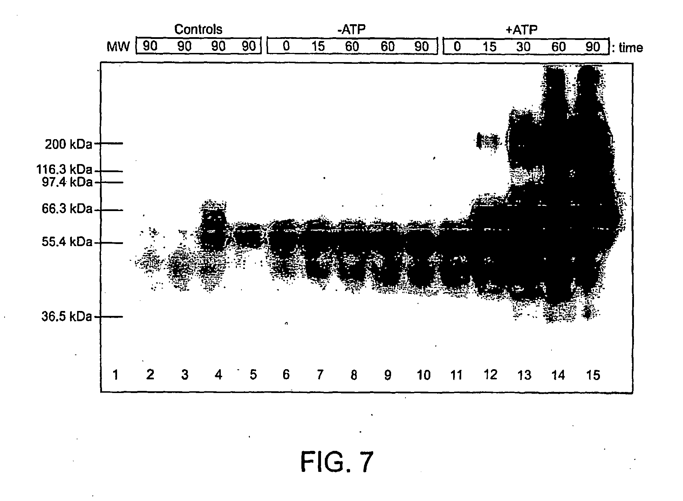

Figure 7 shows the results of an activity assay for purified human Parkin from recombinant *E. coli.* The lanes on the immunoblot show corresponds to (1) MW markers; (2) E1, UbcH7, ubiquitin, time = 90 minutes; (3) E1, UbcH7, ubiquitin, ATP, time = 90 minutes; (4) E1, ubiquitin, Parkin, ATP, time = 90 minutes; (5) UbcH7, ubiquitin, Parkin, ATP, time = 90 minutes; (6) E1, UbcH7, ubiquitin, Parkin, time = 0; (7) E1, UbcH7, ubiquitin, Parkin, time =15 minutes; (8) E1, UbcH7, ubiquitin, Parkin, time = 30 minutes; (9) E1, UbcH7, ubiquitin, Parkin, time = 60 minutes; (10) E1, UbcH7, ubiquitin, Parkin, time = 90 minutes; (11) E1, UbcH7, ubiquitin, Parkin, ATP, time = 0; (12) E1, UbcH7, ubiquitin, Parkin, ATP, time =15 minutes; (13) E1, UbcH7, ubiquitin, Parkin, ATP, time = 30 minutes; (14) E1, UbcH7, ubiquitin, Parkin, ATP, time = 60 minutes; (15) E1, UbcH7, ubiquitin, Parkin, ATP, time = 90 minutes.

### DETAILED DESCRIPTION

### I. Introduction

Genetic data have established that in humans loss of Parkin protein results in the progressive loss of dopaminergic neurons in the substantia nigra and eventually to Parkinson's Disease ("PD"). The relevant Parkin activity in dopaminergic neurons is likely to be its E3 ubiquitin ligase activity. The present disclosure contemplates a therapeutic approach to restore or augment Parkin ligase activity using therapeutic agents, such as small molecules, that can help Parkin achieve or maintain and active conformation. In one aspect, the invention relates methods for identification of agents useful for treating Parkinson's Disease. These methods include cell-based assays.

The disclosure also provides methods for purification of enzymatically active Parkin expressed in recombinant bacterial cells. In a related aspect, the disclosure provides Parkin purified using these methods. The recombinant Parkin can be used in screening assays of the invention as well as for other applications (e.g., to establish standards for Elisa assays, for use as an immunogen to generate monoclonal antibodies, and other uses that will be apparent to scientists and physicians).

These and other aspects of the invention are discussed in more detail below.

### II. Definitions

The terms "Parkin," and "Parkin protein" are used interchangeably and refer to wild-type Parkin or mutant Parkin.

"Wild-type Parkin" refers to human Parkin having the sequence of SEQ ID NO:2. or mouse Parkin having the sequence of SEQ ID NO:4. Wild-type Parkin can also refer to Parkin variants having mutation(s) that do not affect the ligase activity of Parkin and do not confer a different phenotype when expressed in cells. Sequences of nucleic acids and proteins encoding Parkin and other proteins are provided for the convenience of the reader and can also be found in the scientific literature. However, the practice of the invention is not limited to the specific sequences provides. It will be appreciated that variants also can be used in place of the sequences provided.

"Parkin mutant" or "mutant Parkin" refer to a Parkin protein with a sequence that deviates from SEQ ID NO:2 by a substitution, insertion or deletion of one or more residues, and has a different activity or confers a different phenotype than is conferred by wild-type Parkin. When referring to Parkin mutants, conventional nomenclature is used. For example, the R275W mutant has a substitution of tryptophan (W) for arginine (R) at position 275. Generally "Parkin mutant" refers to naturally occurring mutant proteins including, for example, R42P, S167N, C212Y, T240M, R275W, C289G, and P437L.

As used herein, reference to an "agent useful for treating Parkinson's Disease" or "candidate compound for treatment of Parkinson's disease" refers to a compound identified as being more likely than other compounds to exhibit therapeutic or prophylactic benefit for patients with Parkinson's disease, *i.e*., a drug candidate. It will be understood by those familiar with the process of drug discovery that a drug candidate may undergo further testing (e.g., *in vivo* testing in animals) prior to being administered to patients. It will also be understood that the therapeutic agent may be a derivative of, or a chemically modified form of, the drug candidates.

### III. Identification of Agents Useful for Treating Parkinson's Disease

It has been discovered (see Examples below) that over-expression of wild-type Parkin in cells inhibits proteasome activity and can lead to the deposition of large insoluble incisions of Parkin protein. Analysis of brain tissue showed that in PD patients Parkin level appear to be elevated relative to healthy brain tissue and enriched in the insoluble fraction. See Example 5. Without intending to be bound by a particular mechanism, it is believed, based in part on experiments described in the Examples, that the wild-type Parkin protein is prone to misfolding, and that accumulation of misfolded Parkin results in: (1) impairment of proteasome activity, (2) generation of aggresomes containing Parkin and other cell proteins, (3) cell morbidity; and (4) loss of Parkin activity. Loss of Parkin activity is a direct mechanism leading to PD (Kitada et. al., 1998, Nature 392:605-608) and point mutations described in this work may also be related to the loss of function of Parkin leading to disease (Foroud et al., 2003, Ann. Neurology 60:796-801).

Moreover, it has been discovered that agents that stabilize Parkin (*i.e*., maintain Parkin in an active conformation even when over-expressed) or induce proper folding of misfolded Parkin are useful therapeutic agents for treatment of Parkinson's Disease. The present invention provides, *inter alia,* drug screening assays based, in part, on this discovery.

The invention provides cell-based assays for such therapeutic agents.

### A. Cell-Based Assays

As described in Examples 1-3, in cells in which wild type Parkin is over expressed, aggresomes (or "Parkin inclusions") are formed and proteasome activity is decreased. In HEK293 cells in culture, Parkin protein is expressed endogenously at low levels. At this endogenous level of expression, the protein does not detectably affect the proteasome pathway, other than by performing its normal ligase activity. However, when Parkin protein is recombinantly expressed from a cDNA driven by an heterologous promoter (i.e., is expressed at high levels in cells compared to normal endogenous expression) Parkin protein, at least some of which is misfolded and/or insoluble, interferes with proteasome function. It is possible that the proteasome inhibition characteristics of high expression of Parkin levels of expression also occurs at a much lower, undetectable level under normal expression conditions. The slow accumulation of misfolded Parkin protein as an insoluble fraction in brain cells may occur over a long period (e.g., 40-80 years) leading to pathology.

Based in part on this discovery, the invention provides cell-based assays for identifying a candidate compound for treatment of Parkinson's Disease. In one assay of the invention, agents that stabilize Parkin or induce proper folding can be identified by the effect of the agent on aggresome formation and/or proteasome function in a cell.

In one embodiment, the assay includes screening for a candidate compound for treatment of Parkinson's Disease by (a) obtaining mammalian cells expressing Parkin; (b) exposing a cell to a test agent; and (c) comparing proteasome function in the cell exposed to the test agent with proteasome function in similar (control) cell not exposed to the test agent. An increased level of proteasome function in the cell exposed to the test agent compared to the control cell indicates the agent is a candidate compound for treatment of Parkinson's Disease. An exemplary assay is described in Example 6.

As discussed in more detail below, in various embodiments of this assay, the cells used may express wild-type Parkin or mutant Parkin. Preferably the level of expression is higher than the normal level for the particular cell used in the assay. In cases in which recombinant Parkin is expressed in a stably or transiently transfected cell, the expression level will essentially always be higher than normal. This is because endogenous levels of Parkin in cells are low and recombinant expression in which Parkin expression is driven by a heterologous (inducible or constitutive) promoter is comparatively high. Levels of Parkin expression in transfected or non-transfected cells can be measured using routine methods (e.g., immunostaining).

### A.1 Proteasome Function Assays

The effect of an agent on proteasome function in cells can be assessed using any assay of proteasome function. A primary proteasome function is degradation of intracellular proteins. In one embodiment of the assay, Parkin is expressed in a cell that also expresses a reporter-degron fusion protein, and the reporter is used to measure proteasome activity. The fusion protein includes a detectable polypeptide sequence with a degradation signal ("degron") added to the C-terminus (or the N-terminus) of the protein. For illustration and not limitation, an exemplary degron sequence is provided as SEQ ID NO:9. The degradation signal serves to target the polypeptide to the proteasome where the polypeptide is degraded. When the activity of the proteasome is compromised, the levels of polypeptide in the cell increase relative to a cell in which the polypeptide is degraded by a normally functioning proteasome. An increase in protein levels can be detected in a variety of ways.

In one embodiment, proteasome function is assayed in cells using a GFPu reporter system. In the GFPu reporter system, cells that express a green florescent protein (GFP) with a degradation signal added to the C-terminus of the protein are used (see Bence et al., 2001, Science 1552-55; Gilon et al., 1998, EMBD Journal 17:2759-66; SEQ ID NOS:6 and 9). As explained above, when the activity of the proteasome is compromised, the levels of GFP in the cell increase. An increase in GFP levels can be detected in a variety of ways including measuring GFP fluorescence levels in live cells or cell extracts and/or measuring levels of the GFU protein by ELISA, immunoblotting, and the like.

Other similar reporter systems can be used, for example, in which a reporter protein other than GFP is used and/or a different degron is used. See, for example, Dantuma et al., 2000, Nature Biotechnology 18:538-543. A variety of other proteins can be used, including reporter proteins such as Red Fluorescent Protein, Yellow Fluorescent Protein (e.g., Living Colors™ Fluorescent Proteins from Clontech, Mountain View CA), beta-galactosidase, luciferase, and the like. Alternatively, any polypeptide sequences detectable by virtue of an activity (e.g., an enzymatic activity that can be measured), antigenicity (e.g., detectable immunologically), a radioactive, chemoluminescent or fluorescent label, or the like. Degrons are known in the art (see, e.g., Gilon et al., 1998, EMBO Journal 17:2759-66; Sheng et al., 2002, EMBO J. 21: 6061-71; Levy et al, 1999, Eur. J. Biochem. 259:244-52; and Suzuki and Varshavsky, 1999, EMBO J. 18:6017-26).

In one version ("the basic assay") the cell-based assay of the invention involves (a) transiently transfecting GFPu-expressing cells with an expression vector encoding wild-type Parkin (b) contacting a portion of the transfected cells with a test agent, and (c) determining whether the rate of degradation of the GFPu protein is increased, and GFPu levels are reduced in the cells contacted with the test agent compared to control cells not contacted with the test agent. Agents that reduce GFPu levels are candidates for further analysis and therapeutic use. It is expected that at least some agents that decrease GFPu levels do so by stabilizing Parkin structure, reducing the amount of misfolded Parkin.

Cell lines expressing the GFPu reporter are available from the ATCC (e.g., HEK-GFPu CRL-2794). Alternatively, cell lines expressing the GFPu reporter or other reporter-degron fusion proteins can be prepared *de novo* by transforming cells with a plasmid encoding the fusion protein. Any of a variety of cells can be used, including HEK293 cells (ATCC CRL-1573), SHSY-5Y cells (ATCC-2266), COS cells (CRL-1651); CHO cells (ATCC-CCL-61) or other mammalian cell lines. Cells can be stably or transiently transfected. Preferably the cells are stable transfectants for consistency across multiple assays.

In alternative embodiments, the assay can be carried out using cells stably expressing Parkin, and transiently transfected with the reporter-degron protein, or with cells transiently transfected with both Parkin and the reporter.

Expression vectors, methods for transient transfection, and methods for cell culture suitable for the practice of the invention are well known in the art and are only briefly described here. As is well known, expression vectors are recombinant polynucleotide constructs that typically include a eukaryotic expression control elements operably linked to the coding sequences (e.g., of Parkin). Expression control elements can include a promoter, ribosome binding sites, RNA splice sites, polyadenylation sites, and transcriptional terminator sequences. The expression vectors are typically replicable in the host organisms either as episodes or as an integral part of the host chromosomal DNA. Examples of mammalian expression vectors include pcDNA 3.1 (Invitrogen, San Diego, CA); pEAK (Edge Biosystems, Mountain View, CA); and others (see Ausubel et al., Current Protocols In Molecular Biology, Greene Publishing and Wiley-Interscience, New York, as supplemented through 2005). Commonly, expression vectors contain selection markers, e.g., ampicillin-resistance or hygromycin-resistance, to permit detection of those cells transformed with the desired DNA sequences. "Transfection" refers to a variety of art-recognized techniques for introducing foreign nucleic acid (e.g., DNA) into a host cell, including calcium phosphate or calcium chloride co-precipitation, DEAE-dextran-mediated transfection, lipofection, and electroporation. Cell culture techniques are also well known. For methods, see Sambrook et al. 1989, Molecular Cloning: A Laboratory Manual, 2nd Edition, Cold Spring Harbor Laboratory press; and in Ausubel, 1989, *supra.*

### A.2 Cells Expressing Parkin

In a preferred proteasome function assay, cells expressing the reporter-degron fusion protein are transfected with an expression vector expressing Parkin, as described above. In one embodiment the expression vector encodes a wild-type Parkin. For example, the cDNA for human Parkin (NM004562) can be inserted into the HindIII/XbaI sites of the vector pcDNA3.1 (Invitrogen, San Diego CA) for use in this assay.

In another embodiment, an expression vector encoding a Parkin mutant is used. As shown in Example 4, expression of certain Parkin mutants results in inhibition of proteasome function. Proteasome function assays using such Parkin mutants can be conducted as described above for wild-type Parkin, except that cells are transfected with an expression vector encoding a Parkin mutant. This proteasome function assay involves exposing a mammalian cell expressing a mutant Parkin to the test compound; comparing proteasome function in the cell exposed to the test compound and proteasome function characteristic of a cell expressing the mutant Parkin not exposed to the test compound. Exemplary Parkin mutants include S167N, C212Y, T240M, R275W, C289G, P437L (see Table 1). In certain embodiments the Parkin mutant used is R275W, C212Y or C289G. Assays using Parkin mutants can be used as an alternative to, or in combination with, assays using wild-type Parkin.

**Table 1**

| Six Parkin mutations for which heterozygosity is correlated to development of PD | |
|---|---|
| Parkin mutation | Proposed mechanism of pathology |
| S 167N | missense mutation/aggresome |
| C212Y | Dominant gain-of function/aggresome |
| T240M | Loss-of-function |
| R275W | Loss-of-function |
| C289G | Reported to form aggresomes |
| P437L | missense mutation/aggresome |

### A.3 Exposing Cell to Test Agents

As described above, Parken-expressing cells are exposed to a test agent to determine the effect of the agent on proteasome function. Most often, cells expressing a reporter fusion protein (see, e.g., Example 1) are grown and transfected with the Parkin encoding expression construct. The cells are cultured for 1-10 days and then exposed to a test agent. Usually the cells are exposed to a test agent 2 or 3 days after exposure to (or the beginning of exposure to) the agent.

A variety of classes of test agents can be used. For example, a number of natural and synthetic libraries of compounds can be used (see NCI Open Synthetic Compound Collection library, Bethesda, Md; chemically synthesized libraries described in Fodor et al., 1991, Science 251:767-773; Medynski, 1994, BioTechnology 12:709-710; Ohlmeyer et al., 1993, Proc. Natl. Acad. Sci. USA 90:10922-10926; Erb et al., 1994, Proc. Natl. Acad. Sci. USA 91:11422-11426; Jayawickreme et al., 1994, Proc. Natl. Acad. Sci. USA 91:1614-1618; and Salmon et al., 1993, Proc. Natl. Acad. Sci. USA 90:11708-11712). In one embodiment, the agent is a small molecule, e.g., a "chemical chaperone," such as a molecule with a molecular weight less than 1000, and often less than 500.

The duration of the exposure can vary, but will usually be from 1 to 24 hours, and most usually from 4 to 16 hours. Similarly, a variety of concentrations of agent can be tested. It will be appreciated that the concentration will vary depending on the nature of the agent, but is typically in the range of 1nM to 5uM. Typically several different concentrations of test agent are assayed (e.g., 1 nM, 10 nM, 100 nM, 1 µM, 10 µM and 100 µM) along with a zero concentration control.

The proteasome function of a Parkin-expressing cell contacted with test agent can be compared with proteasome function characteristic of a cell expressing Parkin but not exposed to the candidate compound. Typically this is accomplished by conducting parallel experiments using cells exposed to the test agent (at various concentrations) and cells not exposed to the test agent. That is, proteasome function in cells is measured in the presence or absence of compound. Alternatively, proteasome function in test cells can be compared to standard values obtained previously for proteasome function in cells. In another variation, proteasome function is measured in the same cells before and then after addition of the test agent.

At the end of culture period, proteasome function can be measured. For example, in GFPu-expressing cells, GFPu fluorescence and/or GFPu quantity can be measured. Measurements may be quantitative, semiquantitative and/or comparative.

It will be apparent to the reader that modifications of the basic assay can be made. For example, culture plates of various types (e.g., 6; 24, 96, or 384 well plates,) or other high through-put devices can be used can be used for cell culture, optionally in combination with robotic devices, with concomitant adjustment of plasmid quantity in the transfection.

In one embodiment of the invention, HEK293 GFPu cells are grown to 75% density in culture wells of a six-well cell culture plate (e.g., each well approximately 30 mm in diameter). The cells are transfected the Parkin expression vector described above, using approximately 2.5 ug of plasmid per well, and the cells cultured for about 3 days (e.g., 2 to 5 days) prior to analysis with a test agent.

### A.4 Proteasome Function Assays to Determine Whether The Effect of an Agent is Specific for Parkin

Proteasome function assays to establish Parkin specificity can be conducted by using the basic assay described above for wild-type Parkin, except that cells are transfected with an expression vector encoding a different protein believed to be prone to misfolding. For example, the Huntingtin (Htt) protein (SEQ ID NO:11) or CFTR (SEQ ID NO:10; accession number NM000492) proteins may be used. Other proteins prone to misfolding that may be used in this assay include SOD1, Rhodopsin, connexin 43, Ub+1, and presenilin. This proteasome function assay involves exposing a mammalian cell expressing the non-Parkin protein (e.g., Huntingtin) to the candidate agent and comparing proteasome function in the cell with proteasome function characteristic of a cell expressing Huntingtin not exposed to the candidate agent. An agent that stabilizes or increases proteasome function in cells expressing Parkin but not cells expressing Huntingtin or other proteins is likely specifically modulating the effect of Parkin on proteasomes. An agent that stabilizes or increases proteasome function in cells expressing Huntingtin or other proteins as well as in cells expressing Parkin may be acting nonspecifically.

### A.5 Parkin Aggregation Assays

In one embodiment, the assay includes screening for a candidate compound for treatment of Parkinson's Disease by (a) obtaining mammalian cells expressing wild-type or mutant Parkin; (b) exposing a cell to a test agent; and (c) comparing Parkin aggregation in the cell exposed to the test agent with Parkin aggregation characteristic of a control cell not exposed to the test agent. A reduced level of Parkin aggregation in the presence of a test agent indicates the agent is a candidate compound for treatment of Parkinson's Disease. In one embodiment the mammalian cells express wild-type Parkin. In one embodiment the mammalian cells express a mutant Parkin. In some cases the mutant Parkin is S 167N, C212Y, T240M, R275W, C289G, P437L. Preferably R275W, C212Y or C289G is used.

### B. Parkin Binding Assays

An expected characteristic of many chemical chaparones is that they bind to the protein target. Thus, candidate agents useful for treatment of Parkinson's disease can be identified using a Parkin binding assay. The binding assays usually involve contacting purified Parkin protein with one or more test compounds and allowing sufficient time for the protein and test compounds to form a binding complex. Any binding complexes formed can be detected using any of a number of established analytical techniques. Protein binding assays include, but are not limited to, methods that measure co-precipitation, co-migration on non-denaturing SDS-polyacrylamide gels, and co-migration on Western blots (see, e.g., Bennet and Yamamura, 1985, "Neurotransmitter, Hormone or Drug Receptor Binding Methods," in Neurotransmitter Receptor Binding (Yamamura, H. I., et al., eds.), pp. 61-89. The Parkin protein utilized in such assays can be from mammalian cells (recombinant or naturally occurring) or purified Parkin from recombinant bacterial cells.

### C. Parkin Activity Assays

Parkin is an ubiquitin ligase (Shimura et. al., 2000, Nature Genetics 25:302). Ubiquitin ligase activity is defined by the ability of a protein to recognize a specific ligase substrate, and interact with an E2 enzyme to transfer an ubiquitin molecule from the E2 to the substrate. Ligase activity has been shown to be regulated by accessory proteins, but can also occur with the ligase alone (see Joazeiro and Weissman, 2000, Cell 102:549-52).

In one embodiment, an *in vitro* assay used to determine whether a candidate agent is useful for treating Parkinson's disease includes measuring the effect on Parkin ligase activity. In an embodiment the ligase activity is the autoubiquitination activity of a purified Parkin protein in the presence of the compound, and comparing the autoubiquitination activity of purified Parkin protein in the presence of the compound with autoubiquitination activity of purified Parkin protein in the absence of the compound. The ability of an agent to increase autoubiquitination activity is indicative of an agent useful for treating Parkinson's disease and a candidate for further testing. In addition, agents that stimulate autoubiquitination activity may increase the affinity of ligase for substrate, or prevent intracellular turnover of Parkin protein, and are therefore of interest for those activities as well.

Parkin autoubiquitination activity can be assayed in a solution assay or an immobilization assay, as described below and in the Examples.

### C.1 Assays Using Immobilized Parkin

In the immobilization assay, recombinant or purified Parkin is immobilized on a surface (such as a microwell plate, sepharose beads, magnetic beads, and the like) and incubated with a ligase reaction mix that includes ubiquitin. The level of ubiquitination of Parkin under the assay conditions is determined as a measure of Parkin autoubiquitination activity.

Any method for immobilizing Parkin that does not interfere with Parkin activity can be used. In one embodiment, Parkin is immobilized in wells of a 96-well or 386-well microwell plate. Microwell plates are widely available, e.g:; from Immulon (Waltham, MA) and Maxisorb (Life Technologies, Karsruhe, Germany). Parkin can be immobilized using an antibody binding system in which an antibody that recognizes Parkin is immobilized on a surface, and Parkin is added and captured by the antibody. Alternatively the antibody can recognize an epitope tag fused to the Parkin protein (e.g., His, GST, Flag, Myc, MBP, and the like). An antibody is selected that does not interfere with Parkin enzymatic activity. Methods for antibody-based immobilization and other immunoassays are well known (see, e.g., Harlow and Lane, 1988, Antibodies, A Laboratory Manual, Cold Spring Harbor Publications, New York). In an other approach, Parkin protein with a N-terminal His₆ tag can be immobilized using a nickel-coated assay plate.

After a blocking step, a ligase reaction mix including E1 (ubiquitin-activating enzyme, optionally epitope tagged, as with GST or His₆), E2 (ubiquitin conjugating enzyme), ATP-Mg, and ubiquitin (usually labeled ubiquitin) is combined with immobilized Parkin (Parkin E3 ligase). Purified ubiquitin pathway enzymes can be obtained commercially (e.g. from Boston Biochem Inc., 840 Memorial Drive, Cambridge, MA 02139) or prepared as described in Wee et.al., 2000, J. Protein Chemistry 19:489-498). Blocking to reduce nonspecific binding of E1 to the plate can be with SuperBlock (Pierce Chemical Company, Rockford, IL); SynBlock (Serotec, Raleigh, NC); SeaBlock (CalBiochem, Darmstadt, Germany); metal chelate block (Pierce Chemical Company, Rockford, IL); 1% casein; glutathione; and various combinations of these, with 1% casein preferred in some embodiments. After the blocking step, the wells can be washed with SuperBlock wash (Pierce Chemical Company, Rockford, IL) or Ligase buffer wash (50mM HEPES/ 50mM NaCl). In one embodiment, Immulon 96 or 384 well plates are blocked with 1% casein in 50mM HEPES/50mM NaCl and washed using 50mM HEPES/50mM NaCl/4mM DTT.

An exemplary reaction mix is:

| | |
|---|---|
| 1:1 Biotin:ubiquitin | 500nM |
| GST-E1 | 2-6nM |
| E2 (UbcH7) | 300nM |
| *E. coli* Parkin protein | 2-10ug |
| MgATP | 10mM |
| Buffer | 50mM HEPES/50mM NaCl/pH 8.8 |

*E. coli* Parkin protein can be prepared as described below in Section IV. The assay can be carried out at 37°C for 1 hour and stopped by washing wells with 50mM HEPES/ 50mM NaCl. ATP can be omitted from certain samples as a negative control. In one embodiment, the assay carried out in a 96 or 384 well plate format. The plate is incubated for a period of time (e.g., 60 minutes at room temperature or 40-60 minutes at 37°C). Plates are washed to remove soluble reagents and the presence or amount of ubiquitin (*i.e*. the ubiquitin component of autoubiquinated Parkin) is determined.

Methods for detection of ubiquitin will depend on the label or tag used. For example, in a plate assay, fluorescein-tagged ubiquitin, can be detected directly using a fluorescence plate reader, biotin-tagged ubiquitin can be detected using labeled strepavidin (*e.g*., strepavidin-HRP or 1:5000 Neutravidin-HRP [Pierce Chemical Comp. Rockford, IL]), and epitope-tagged ubiquitin can be detected in an immunoassay using anti-tag antibodies. Epitope tags are fused to the N-terminus of ubiquitin or otherwise attached in a way the does not interfere with ubiquitination. These assays and other useful assays are well known the in art.

### C.2 Assays Using Parkin in Solution

In an alternative approach, the autoubiquitination assay for Parkin is carried out in solution and then the solution (or an aliquot) is transferred to a capture plate for quantitation. In an exemplary reaction, the reaction components (below) are assembled in 50 microliter volume and the assay is run for from 10 to 90 minutes (e.g., 60 minutes) at 37°C.
Reaction components:
50mM HEPES/50mM NaCl/pH 8.8
500nM 1:1 Biotin: ubiquitin
2-6nM GST-E1
300nM E2 (UbcH7)
2- 10ug *E. coli* recombinant Parkin protein
10mM MgATP*
*Can be added last to initiate the reaction.
After reaction is complete, the reaction mix is transferred to a capture plate (e.g., 96 or 384 well plate) containing an immobilized moiety that binds Parkin (e.g., anti-Parkin antibody, nickel for His-tagged Parkin, or anti-epitope tagged antibody such as anti-flag GST, His, Myc, MBP, etc. for epitope-tagged Parkin). When nickel plates are used to immobilize His-tagged Parkin the reaction may be stopped by the addition of 6M Guanidinium HCl. This capture plate can be blocked with 1% casein. The reaction mix is incubated in the capture plate for 60 minutes. After this time, the plate is washed 3x with 50 mM HEPES/50mM NaCl/4mM DTT). Detection is carried out using a reagent that binds to the tag present on the ubiquitin moiety (e.g., strepavidin-HRP) and processed using standard procedures.

### D. Combinations of Assays

The cell-based and protein-based assays described above can be used independently or in various combinations to identify candidate compounds for treatment of Parkinson's Disease that reduce proteasome impairment in cells expressing Parkin proteins. In one embodiment, the "basic assay" for agents that ameliorate the inhibition of proteasome function in cells expressing wild-type Parkin can be used in combination with additional assays such as: (1) proteasome function assays using Parkin mutants (2) Parkin aggregation assays (3) proteasome function assays to establish Parkin specificity (4) Parkin binding assays (5) *in vitro* protein activity assays. When used in combination, these assays can be conducted in any order. For example, initial high-throughput screening can be conducted using an *in vitro* protein assay and the basic cell-based assay can be used as a secondary screen. Alternatively, for example, cell-based assays can be conducted first and *in vitro* protein binding and activity assays can be used as a secondary screen. Other sequences and combinations of assays will be apparent to the reader.

In one embodiment agents that rescue proteasome function both in cells expressing wild-type Parkin and in cells expressing a mutant Parkin are identified as particularly promising drug candidates and subjected to further testing. In one embodiment agents are selected that rescue proteasome function in multiple cell lines, such as cells expressing proteins selected from wild-type Parkin and mutant Parkins (e.g., R275W, C212Y and C289G).

In one aspect of the invention, combinations of different cell-based assays and protein based assays are used to screen for agents useful for treatment of Parkinson's disease. For example, the basic cell based assay using wild-type Parkin can be using in conjunction with any one or combination of assays described above. Solely for illustration and not for limitation exemplary combinations of assays (and exemplary, non-limiting, profiles of agents considered useful) are shown in the table below. For example, one screening approach (C) comprises two assays: the cell based assay with wild-type Parkin and the Parkin activity assay. These assays can be conducted in any order.

**TABLE 1**

| | A | B | C | D | E | F |
|---|---|---|---|---|---|---|
| 1. Basic cell based assay with wild-type Parkin | + | + | + | + | + | + |
| 2. Basic cell based assay with mutant Parkin | + | - | | | + | |
| 3. Specificity assay | ++ | ++ | | | | ++ |
| 4. Protein binding assay | * | | | | | |
| 5. Protein activity assay | ** | | ** | | | |

| | | | | | | |
|---|---|---|---|---|---|---|
| + Increases proteasome function - Does not increase proteasome function ++ Does not increase proteasome function in nonParkin expressing cells * binds ** increases ligase activity | | | | | | |

### IV. Expression and Purification of Enzymatically Active Recombinant Parkin

Wild-type and mutant Parkin proteins can be expressed and purified from recombinant mammalian cells (e.g., pcDNA-Parkin expressing vectors stably integrated into HEK-293 cells). Alternatively, recombinant Parkin can be obtained using Baculovirus expression or bacterial expression. However, heretofore, techniques that result in efficient purification of enzymatically active Parkin expressed in bacterial cells (e.g., *E. coli*,) have not been described.

### A. Expression of Recombinant Parkin Protein

Parkin protein can be produced by expression in *E. coli* and other prokaryotic hosts using routine methods of transformation, selection, and culture. Exemplary *E. coli* strains that may be used include BL21; BL21-pLysS; BL21-Star; BL21-Codon+; BL21(DE3); BL21(DE3)-Star; L21(DE3)-Codon+; and BL21-A1. Other useful bacterial expression systems include bacilli (such as Bacillus subtilus), other enterobacteriaceae (such as Salmonella, Serratia, Pseudomonas aeruginosa, and Pseudomonas putida) or other bacterial hosts (e.g., Streptococcus cremoris, Streptococcus lactis, Streptococcus thermophilus, Leuconostoc citrovorum, Leuconostoc mesenteroides, Lactobacillus acidophilus, Lactobacillus lactis, Bifidobacterium bifidum, Bifidobacteriu breve, Bifidobacterium longum, and Yersinia pestis).

Parkin can be expressed as a fusion protein with an affinity or epitope tag to facilitate purification. Exemplary tags include glutathione S-transferase (GST), dihydrofolate reductase (DHFR), maltose binding protein (MBP), 6x histidine [His]₆, chitin binding domain (CBD), and thioredoxin. A preferred tag is one that can be bound by an affinity ligand under denaturing conditions, e.g., in the presence of 6M guanidinium hydrochloride (GuHCl). A preferred tag is poly-histidine (e.g., [His]₆). Example 1 describes expression of full-length His-tagged Parkin for purification using nickel-mediated affinity chromatography.

### B. Purification and Refolding of Recombinant Parkin Protein

The inventors have discovered that when expressed in *E. coli,* Parkin partitions to inclusion bodies. Enzymatically active Parkin, particularly His₆-tagged Parkin, can be recovered from inclusion bodies of *E. coli* and other prokaryotes using a four-step process involving:
1) Purification of inclusion bodies
2) Disruption of inclusion bodies
3) Chromatography
4) Refolding.
Each of these steps is described below.

### B.1 Purification of Inclusion Bodies

A variety of methods are known for purification of inclusion bodies and are suitable for practice of the present disclosure See, e.g., Current Protocols in Protein Science (2003) Ch. 6: "Preparation and extraction of insoluble (inclusion-body) proteins from Escherichia coli*."*

### B.2 Disruption of Inclusion Bodies

A variety of methods are also known for disruption of inclusion bodies. *See,* e.g., Current Protocols in Protein Science, supra and Clark, 1998, Current Opinion in Biotechnology 9:157-163. In a preferred embodiment of the invention, inclusion bodies are disrupted using guanidine hydrochloride (e.g., 2 to 6 M GuHCl) and a reductant (e.g., 1 to 10 mM DTT; 4 mM TCEP [Tris (2-carboxyethyl)phosphine hydrochloride]; 4-10 mM beta-mercaptoethanol, and the like). For example, an inclusion body pellet can be combined with 5-10 volumes Suspension Buffer [50mM HEPES, pH 8.5, 6M GuHCl, 10 mM beta-mercaptoethanol] and disrupted using a dounce homogenizer, sonication or other methods. Following disruption, any remaining insoluble material can be removed by centrifugation and/or filtration. The resulting soluble fraction contains Parkin and is suitable for affinity chromatography as described below.

In alternative embodiment, denaturants other than guanidinium hydrochloride can be used for disruption of inclusion bodies. In one alternative embodiment guanidinium isothiocyanate (e.g., 2-6 M) can be used. In other, less preferred, embodiments denaturants such as urea [2-8M]; sarkosyl(N-lauroylsarcosine) [1-2%]; Triton X-100 + sarkosyl [0.5-2% + 1-2%]; N-cetyl trimethylammonium chloride [2-5%]; N-octylglucoside [0.5-2%]; sodium dodecyl sulfate [0.1-0.5%]; alakaline pH to ph >9 (e.g., addition of NaOH); combinations of the foregoing; and other denaturants. See, Purification Handbook, Amersham Pharmacia Biotech p.71 (1999). Generally, the washed inclusion bodies are resuspended in the denaturants for 1-60 minutes (depending on the particular preparation, as well as the quantity of inclusion bodies being solubilized). It will be recognized that, in some cases (e.g., 8 M urea) the denaturant usually will be at least partially removed prior to affinity chromatography.

### B.3 Affinity Chromatography.

The nature of the affinity chromatography used will depend on the tag used. As noted, the affinity interaction between the solid phase (affinity resin) and Parkin fusion protein should be stable in high concentrations of guanidinium hydrochloride (e.g., 2 to 6 M GuHCl). In one embodiment, immobilized metal affinity chromatography (IMAC) is used. IMAC exploits the ability of the amino acid histidine to bind chelated transition metal ions, e.g., nickel (Ni²⁺), zinc (Zn²⁺), copper (Cu²⁺) or cobalt (Co²⁺). Usually nickel or cobalt is used. Immobilized nickel products for use in chromatography are readily available (e.g., Ni-NTA resins (Qiagen, Inc)). Immobilized cobalt products for use in chromatography are readily available (e.g., HIS-Select™ Cobalt Affinity Gel; Sigma-Aldrich Corp.).

Following application of the soluble fraction to the affinity column, the column is washed to remove unbound material and the Parkin-His₆ fusion protein is eluted. Conveniently fusion protein can be eluted using imidazole (e.g., 100-500mM). In one embodiment, the elution buffer is 50 mM HEPES, pH 8.0, 5.5M GuHCl, 500 mM imidazole, 10 mM beta-ME, 0.5 mM EDTA. Fractions containing the target protein are recovered. Optionally, reductant can be added as fractions are collected (e.g. to increase the concentration of reductant can be increased the collected fractions (e.g., to 19 mM beta-ME).

### B.3 Refolding

Two dialysis steps are used for recovery of active Parkin from the GuHCl-containing solution. In the first dialysis step, the eluted material is dialyzed against a buffered solution containing arginine and a reducing agent. Arginine may be present in the range 0.1 to 1 M, such as in the range 0.2 to 0.8 M, 0.3 - 0.6 M or 0.35-0.5 M). Exemplary reductants include DTT (e.g., 1 to 10 mM, e.g., 10 mM); Tris (2-carboxyethyl)phosphine hydrochloride (TCEP, e.g., 4mM TCEP); beta-mercaptoethanol (e.g., 4-10 mM beta-mercaptoethanol), and agents with similar reducing power. An exemplary buffer is 0.4 M arginine, 50 mM HEPES, pH 8.0, 10 mM DTT.

In the second dialysis step the dialysate from step one is dialyze against a buffer that is substantially free of arginine. Any buffer in which proteins generally are stable (i.e., in which most proteins retain their structure and activity) can be used, so long as the buffer contains at most minimal amounts of arginine (i.e., less than 0.5 mM, preferably not more than 0.1 mM, most preferably no arginine). An exemplary buffer is 50 mM HEPES, pH 8.0, 0.2M NaCl, 10 mM DTT. Optionally, the glycerol can be added to the sample before this dialysis step. Additional dialysis steps can be carried out, if desired.

The dialysate containing Parkin can be collected and any precipitant removed. The activity of the purified protein can be determined using an autoubiquitination assay. See Lorick et al., 1999, "RING fingers mediate ubiquitin-conjugating enzyme (E2)-dependent ubiquitination" Proc Natl Acad Sci USA 96:11364-9 and Example 10. Preferably the specific activity of the purified Parkin material (>95% Parkin protein) is at least about 0.1 Unit/0.5 microgram Parkin protein. For example, the specific activity may be between 0.1 Unit per 0.5 micrograms and 5 Units/0.5 micrograms. In certain embodiments the specific activity is at least about 0.2 U, at least about 0.25 U, or at least 0:5 U/0.5 microgram Parkin protein In referring to specific activity a "Unit" is defined as the ability of a Parkin protein preparation to transfer 50 ng ubiquitin to Parkin in 15 minutes (e.g., under the assay conditions described in Example 10, below, where from 0.5 to 10 micrograms, usually 0.5 micrograms, Parkin protein is used in the reaction) or, equivalently, one-quarter unit is the ability to transfer 25 ng ubiquitin to Parkin in 30 minutes. His-tagged Parkin prepared according to the Examples, below, had a specific activity of approximately one-quarter Unit per 0.5 microgram Parken (i.e., 25.2 ng ubiquitin transferred in 30 minutes). Alternatively, Parkin activity can be demonstrated using any assay that measures an enzymatic activity and/or biological function of Parkin.

The purified Parkin protein can be used in a variety of applications, including screening assays, immunological assays, assay standards and others. The Parkin protein can be modified (e.g., conjugated to other compounds and/or an epitope tag removed) or processed as necessary for a particular application. In some embodiments the His epitope tag is removed. For example, the plasmid pET30a vector described in Example 7 the His-tag can be removed by digestion with thrombin or enterokinase (see SEQ ID NO:5).

### V. EXAMPLES

### Example 1: GFPu HEK293 cells

GFPu-expressing cell lines were prepared as follows: HEK293 cells (ATCC No. CRL-1573) were transformed using a construct in which an oligonucleotide encoding a short degron (Gilon et al., 1998, EMBO Journal 17:2759-66) is inserted C-terminal to coding sequence for GFP (Heim et al., 1994, Proc. Nat. Acad. Sci. USA 91:12501-504; Accession #P42212). Cells were transfected with 2ug cDNA. The cells were cultured for 48 hours and transformants were selected using 1000 ug/ml G418 (geneticin). After an additional 7 days, the cell growth media (DMEM plus 1000ug/ml G418) was changed by removing old media and adding fresh media. Cells were allowed to grow for two weeks to select for cells that were resistant to G418. These cells were then collected and sorted by FACS techniques to identify and isolate single cells. These single cells were individually sorted into 96-well plates and allowed to grow and proliferate over two week period. The cells were then plated into duplicate 96 well plates. One plate was analyzed by FacScan the other plate was used to expand clones that were identified as positive in the FacScan analysis.

Clones were screened for very low background levels of GFP and an increase of more than 2 log units of fluorescence in the presence of the proteasome inhibitor epoxomicin. Two GFPu expressing cell lines, lines 60 and 61, were used in the remainder of the experiments.

Cells from the two GFPu cell lines were grown to 75% density in six-well plates, transfected with 2.5 ug per well of cDNA expression vectors encoding Parkin, Parkin mutants, Synuclein, or Synuclein variants. The cells were cultured for 2-5 days and examined using fluorescence microscopy and FACScan to measure GFP fluorescence. In some cases, epoxomicin was added 5 hours prior to FACScan as a positive control for GFPu levels. In addition, cell extracts were prepared for immunoblotting.

### Example 2: Expression of Wild-Type Parkin Results in Parkin Inclusions and Decreases in Proteasome Activity In GFPu-Expressing Cell Lines.

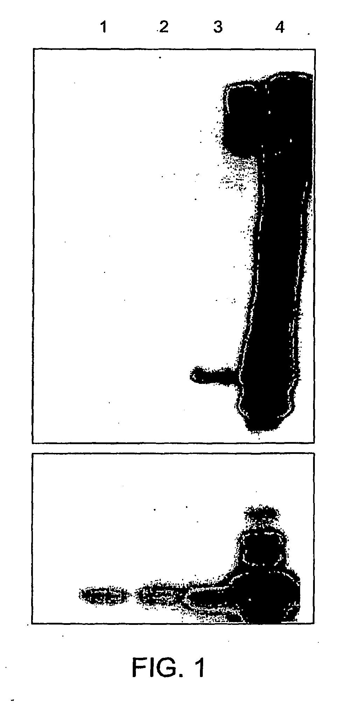

In both of the GFPu cell lines (lines 60 and 61), the overexpression of Parkin resulted in formation of Parkin aggregates, as determined by immunoblotting and microscopy. Parkin transfection also resulted in a striking increase in GFPu levels, indicating that expression or overexpression of Parkin impaired proteasome activity. Figure 1 shows an immunoblot from cell line 60. GFPu/293 cells were transfected with pcDNA3.1 vector (lanes 1 and 2) or with pcDNA3.1-Parkin (lanes 3 & 4). 48 hours post transfection, cells were extracted for soluble protein and insoluble protein and these extracts were analyzed by immunoblotting for GFPu (bottom panel) or Parkin (top panel). Soluble protein extract (lanes 1 and 3); insoluble protein extract (lanes 2 and 4). These data demonstrate a clear accumulation of GFPu protein after Parkin expression (compare lanes 3 & 4 with lanes 1 & 2), and also demonstrate the distribution of GFPu protein into the insoluble protein fraction after Parkin overexpression (compare lane 4 with lane 3).

### Example 3: Overexpression of Parkin, But Not Synuclein, Results in Aggresomes

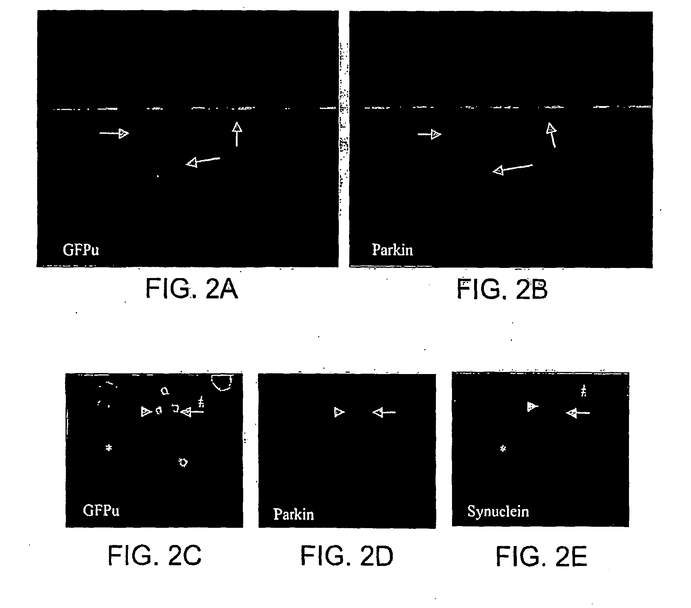

Figure 2 shows epifluorescent and immunofluorscent images illustrating that expression of Parkin protein leads to stabilization and aggregation of other proteasome substrates such as GFPu. Parkin cDNA was transfected in to GFPu 293 cells prepared as described in Example 1 alone (Panels A and B) or with cDNA for alpha-synulein and Parkin cDNA (Panels C, D and E). The cells were fixed after 48 hours and processed for immunofluorescence microscopy. Parkin protein was localized by staining with antibody HPA1A to residues 85-96 of human Parkin protein, alpha-synuclein was localized by staining with Syn-1 antibody (Transduction labs, San Jose, CA), and GFPu was localized based on the green fluorescence of the protein.

In cells expressing Parkin, Parkin protein (Panel A, arrows) is found as aggregates in the cells, and is colocalized with aggregates or accumulation of GFPu (Panel B, arrows). In cells not expressing Parkin protein (asterisk) there was no accumulation of GFPu.

In cells expressing both Parkin and alpha-synuclein, alpha-synuclein does not aggregate and is not required for the Parkin-mediated increase in GFPu. Arrows show that in cells expressing both synuclein and Parkin, aggregates of Parkin (Panel C) and GFPu (Panel D), but not of alpha-synuclein (Panel E), are found. Arrowhead indicates cells expressing only Parkin. The # symbol identifies a cell expressing alpha synuclein but not Parkin. This cell does not have an increase in GFPu, indicating synuclein is not sufficient to increase GFPu. It is clear from this that the GFPu is accumulated/aggregated in cells expressing Parkin, and alpha-synuclein is not required.

### Example 4: Expression of Mutant Parkins Heterozygous "Dominant" Parkin mutations

Expression plasmids encoding (1) wild-type Parkin or (2) mutant Parkin for which heterozygosity is correlated to development of PD were transfected into HEK 293/GFPu cells to assess the effect of the mutant Parkin proteins on proteasome function and aggregation (see Table 1).

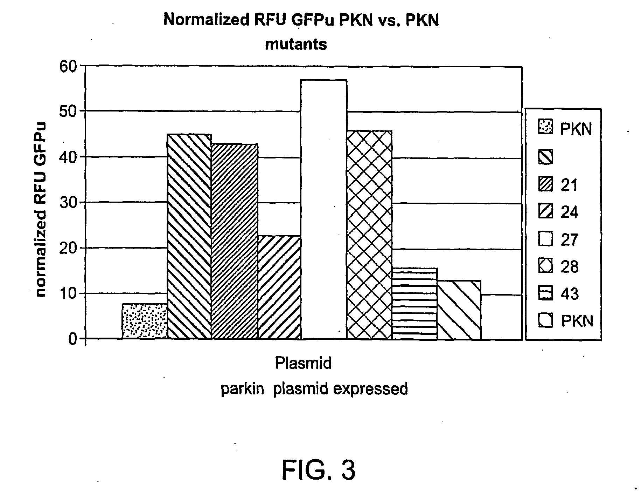

Figure 3 shows the results of FACscan analysis 2 days post transfection. Inhibition of proteasome activity was significantly higher with mutants S 167N, R275W, C212Y and C289G than for wild-type Parkin. Mutants R275W, C212Y and C289G significantly reduced proteasome activity at all times and transfection concentrations tested.

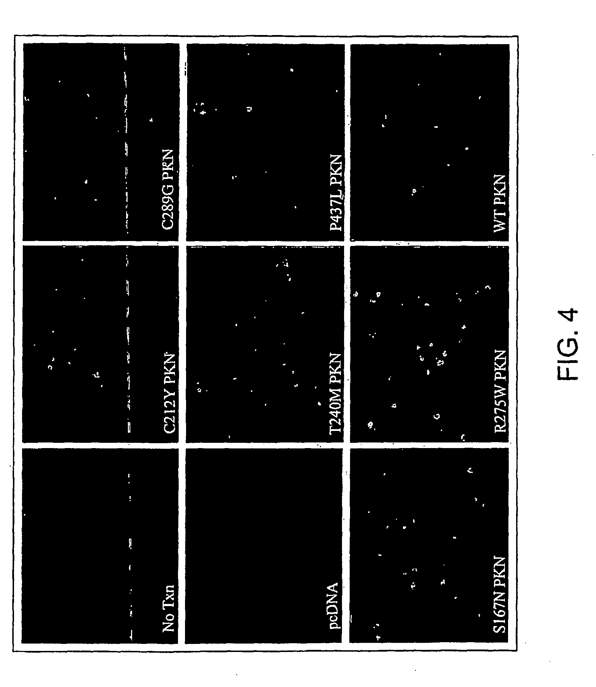

Figure 4 shows epifluorescence images of each sample five days after transfection of the HEK 293/GFPu cells. The images were recorded using the same camera settings for each sample to reflect the level of fluorescence intensity, a direct measure of GFPu levels in the cells. As shown in the figure, expression of Parkin mutants can force GFPu into an aggresome. As shown in Figure 4, and confirmed in experiments using an ArrayScan^{®} high content screening device (data not shown), expression of mutants S167N, R275W, C212Y and C289G increased GFP levels (*i.e*., significantly reduced proteasome activity).

### Example 5: Parkin Distribution in Human Brain Tissue

The location and characteristics of Parkin protein in human brain tissue from sporadic PD patients and healthy controls was determined by immunoblotting of brain extracts.

Methods: Brain tissue from sporadic PD and normal individuals was obtained from the UCLA brain bank. Each sample consisted of tissue from four brain regions: Frontal cortex, caudate nucleus, putamen and substantia nigra. The later three brain regions are components of the nigrostriatal pathway. Frozen brain tissue from each brain region was homogenized via dounce, and extracted at a ratio of 0.5mg tissue/1ml of IPB extraction buffer (50mM tris 7.5; 300mM NaCl; 0.05% Deoxycholate; 0.1 % NP-40, 5mM EDTA). After 20 minutes on ice, homogenates were spun for 10 minutes at 10,000 x g. This supernatant was removed and the pellet was extracted again in the same manner, and centrifuged again. This second IPB supernatant was removed and the final remaining pellet was then solubilized in 1% SDS/10m Tris 7.5 for ten minutes at room temperature, followed by sonication for 20 seconds.

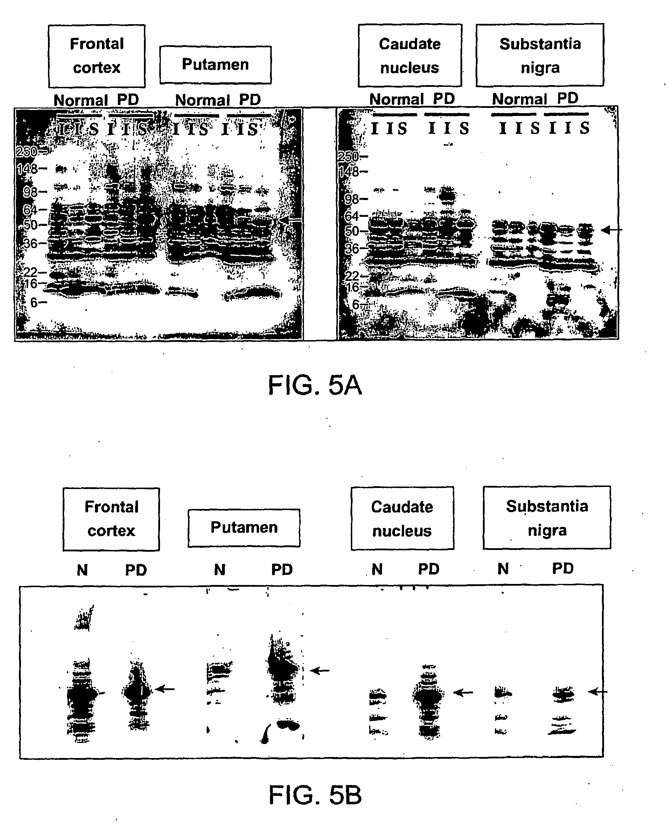

Figure 5 shows the distribution of Parkin protein in normal and PD brain. Brain protein was seperated electrophoretically and immunoblotting was carried out using HPA1A, a polyclonal antibody to human Parkin residues 85-95. In Figure 5A (I) is the IPB fraction and (S) is the SDS fraction, as described above. It is noteworthy that in the PD samples, the amount of 52-kD Parkin protein overall is increased, and the amount of insoluble Parkin is also increased.

A direct comparison of insoluble material from the same samples is provided in Figure 5B. Compared to Figure 5A, there is an accumulation of higher molecular weight material, possibly because the samples were sonicated just prior to loading the Figure 5B gels, but not the Figure 5A gels. Although Parkin is increased in the frontal cortex of both samples, it is increased in the nigrostriatal portions of the brain (putamen, caudate nucleus and the substantia nigra) only in the PD patients. The data in Figure 5 suggest that in sporadic PD patients, Parkin levels may be increased relative to controls overall and enriched in the insoluble fraction. Insoluble protein is highly unlikely to be active.

### Example 6. Cell Based Assay

Hek293 cells stably expressing a proteasome-targeted Green Fluorescent Protein (GFP) were transiently transfected with an expression vector expressing wild type Parkin or with a vector only control (pEAK; Edge Biosystems, Mountain View, CA). The cells were maintained at 37°C in 5% CO₂ for 16-24 hours. Cells were subsequently fixed with 3.7% formaldehyde and then washed 2x with PBS. Cells were then stained with 1 ug/ml Hoechst dye for 15 minutes at room temperature and then washed 2x with PBS leaving 200 ul of PBS in the well. Cells were imaged on the ArrayScan VTI using the XF100 filter set that had been optimized for GFP. Data were collected from at least 200 cells/well. The Target Activation Bioapplication program was used to analyze intracellular fluorescence ("mean average intensity") where the mask modifier was set at 2 pixels.

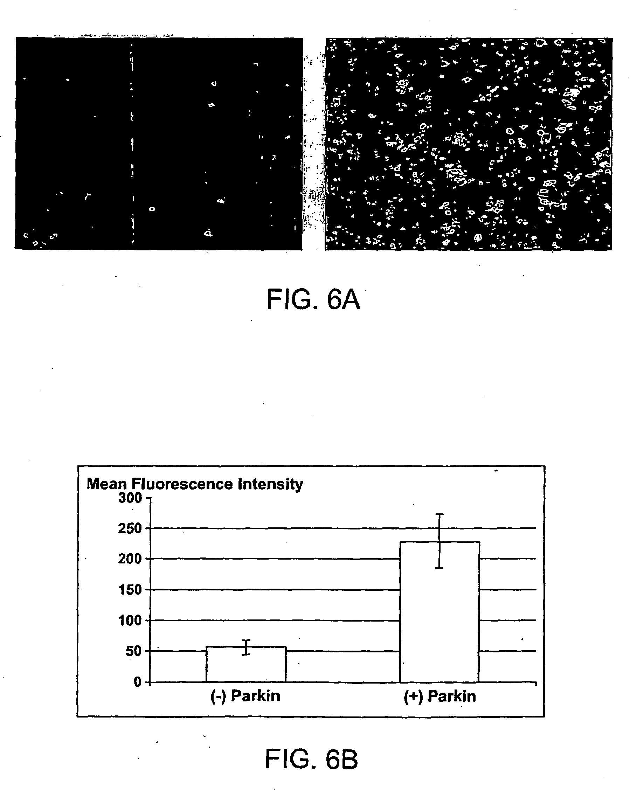

Figure 6A shows fluorescent images of cells transfected with wild type Parkin (upper right) or vector control (upper left). In control transfected cells (vector alone) there was very little fluorescent intensity while in Parkin transfected cells exhibited a marked increase in GFP fluorescence intensity indicative of aggresome formation.

Similar results were observed in a number of different experiments. The signal to background ratio is consistently 3 to 5, where signal is defined as the mean fluorescence intensity from Parkin-transfected cells and background is mean fluorescence intensity from control treated cells (Figure 6B).

Because the cells are transiently transfected with DNA, we confirmed that there were not large variations in measured fluorescence intensities from well to well. Cells in each well of a 96-well plate were transfected with wild type Parkin and the mean average fluorescence intensity from each well was recorded. The coefficient of variation (CV) across the plate was quite low indicating the screening assay provides reliable consistent results.

A particular cell-based assay for identifying a candidate compound for treatment of Parkinson's Disease can be carried out as follows. Hek293 cells stably expressing a proteasome-targeted Green Fluorescent Protein (GFP) are obtained and are transiently transfected with an expression vector expressing wild type Parkin ("test cells"). Vector only control cells are also obtained. Four equivalent subcultures are prepared from the vector only cells and 16 test subcultures are obtained from the each parent culture. A test agent ("TA#100") dissolved in culture medium. The test cells are provided with fresh culture medium containing 0, 1, 10, or 100 micrograms TA#100 and cultured under conditions in which Parkin is expressed. After 2 days the cells are fixed and processed as described above. The cells are imaged on the ArrayScan VTI using filters optimized for GFP. Data were collected from at least 200 cells/well. The fluorescence intensity and distribution in cells exposed to various amounts of TA#100 is determined, the fluorescence intensity being a measure of proteasome function in the cells. A decrease in fluorescence in the presence of TA#100 is evidence of an increased level of proteasome function in the cell exposed to the test agent and indicates the agent is a candidate compound for treatment of Parkinson's Disease. Additional assays are carried out to determine the dose-responsiveness of the effect. It will be appreciated that this example is for illustration and the reader guided by this specification will appreciate that there are numerous variations of this particular assay.

### Example 7: Expression and Purification of Recombinant Human Parkin

This example describes the expression and purification of a human Parkin-oligohistidine fusion protein (i.e., His₆-Parkin). Example 8 describes refolding denatured protein to obtain an enzymatically active product. Use of high concentration arginine and a strong reductant was effective in refolding denatured recombinant Parkin to produce active enzyme. Example 9 describes an optional additional chromatography step that may be used in purification.

### A. Purification of Parkin-containing inclusion bodies.

A sequence encoding full-length human Parkin fused to a histidine tag (see SEQ ID NO:5) was cloned into the bacterial expression plasmid pET30a (Novagen, Madison, WI 53719) to produce pET30a-Parkin. The N-terminal His tag is encoded by the pet30a vector and is fused in frame N-terminal to Parkin when a PCR fragment of Parkin (NM004562) with BamHI/HindIII restriction sites added to the 5' and 3' ends respectively is inserted into a pet30a vector also cut with BamHI/HindIII. *E. coli.* strain BL21 (DE3)-pLysS were transformed and transformants were selected based on antibiotic resistance on LB plates with kanamycin.

10 ml overnight cultures of BL21 (DE3)-pLysS with pET30a-Parkin (grown in LB with 1% glucose, 25 ug/mL kanamycin and 35ug/mL chloramphenicol) were used to inoculate four flasks containing 1 liter each of LB + antibiotics. Cultures grew to an OD₆₀₀ of 0.55-0.6. To induce Parkin expression, IPTG was added to 0.4 mM and the flasks were returned to the shaker and grown at 37C for 4 hours. Cultures were collected by centrifugation (total pellet wet weight=15.6g) and then frozen at -20C overnight.

The frozen pellets were resuspended in 140mLs of Lysis Buffer (50mM HEPES, pH 8.0, 500mM NaCl, 1mM EDTA, 10 mM beta-mercaptoethanol) and homogenized for 3 minutes to break up DNA. The resulting viscous solution was passed through a nebulizer 4 times. The resulting solution was cleared by centrifugation for 20 minutes at 30k RCF (SS34 rotor) and the pellets (containing inclusion bodies) were recovered.

The inclusion bodies were suspended in 200ml Wash Buffer #1 [50mM HEPES, pH 8.0, 500mM NaCl, 1% Triton X-100, 10 mM beta-ME] using the homogenizer for 2 minutes to disperse the suspension. The supernatant was saved for analysis and the inclusion bodies re-pelleted by centrifugation for 20 minutes at 30k RCF.

The inclusion bodies were suspended in 200ml Wash Buffer #2 [50mM HEPES, pH 8.0, 1.0M NaCl, 10 mM beta-ME], again using the homogenizer to disperse the solids. The supernatant was saved for later analysis and the inclusion bodies were repelleted by centrifugation for 20 minutes at 30k RCF. The weight of inclusion body sample was 2.45 g.

The inclusion bodies were resuspended in 20ml of Suspension Buffer (50mM HEPES, pH 8.5, 6M GuHCl, 10 mM beta-ME) using the dounce homogenizer to break apart the solid mass. The sample, which was very dark brown in color, was left overnight at 4°C. The next morning, the remaining insoluble material was removed by centrifugation for 20 minutes at 30k RCF and the supernatant was filtered through a 0.2 µM Tuffryn filter prior to use. 24.5 mls of supernatant was collected, with a protein concentration of 15.9 mg/ml.

### B) Affinity Purification of Parkin Fusion Protein

The sample above was loaded onto a 40mL IMAC column previously charged with nickel sulfate. The chromatography buffers were:
Buffers A: 50mM HEPES, pH 8.0, 5.5M GuHCl, 10 mM beta-ME
Buffer B: 50mM HEPES, pH 8.0, 5.5M GuHCl, 500mM imidazole, 10 mM beta-ME pH was rechecked after all additions are made (except beta-ME, which was added fresh immediately prior to use.) The sample was loaded at 2 ml/min using 1% Buffer B with a total of 2 column volumes used to load the sample and wash the column. An additional wash at 4 ml/min using 5% B for column volumes 2.5 column volumes. 10 mL fractions were collected.

The sample was eluted at 4 ml/min using 2 column volumes of 100% Buffer B. 10 mL fractions were collected. As each column was collected additional beta-ME was added to 20 mM final concentration and 0.5M EDTA was added to 0.5 mM final concentration. Protein concentration was monitored during washing and elution and four 10-mL fractions collected during the elution step were pooled ("Pool 3"). The protein concentration of Pool 3 (50 mM HEPES, pH 8.0, 5.5M GuHCl, 500 mM imidazole, 20 mM beta-ME, 0.5 mM EDTA) was 2.22 mg/ml.

### Example 8. Refolding Denatured Recombinant Parkin to Produce Active Enzyme

Pool 3 from Example 7 was diluted to a protein concentration of about 1.0 mg/ml with (50 mM HEPES, pH 8.0, 10 mM DTT) and 2.X 1 mL samples were dialyzed at 4C overnight against 500mls (1.5M GuHCl, 50 mM HEPES, pH 8.0, 10mM DTT) using 10k MWCO dialysis tubing. No visible precipitation was evident the following morning. One of the samples was dialyzed overnight at 4C against (0.4M arginine, 50 mM HEPES, pH 8.0, 10 mM DTT). No apparent precipitation was apparent. The dialysate was collected and cleared by filtration.

Arginine was removed by further dialysis of the sample overnight at 4C. Sample 8B (500 uL at 1110 ug/mL) was made to ~10% glycerol, then dialyzed against 1000 volumes 50 mM HEPES, pH 8.0, 0.2M NaCl, 10% glycerol, and 10 mM DTT. The following morning, no precipitate was visible in either sample. The samples were centrifuged for five minutes at top speed in a microfuge, then assayed for protein concentration. The recovery for Sample 8B was 78%:

### Example 9 Optional SEC Purification

His₆-tagged Parkin was isolated from inclusion bodies as described in Example 6, section A. GuHCl-solubilized fractions were stored at -80°C and quickly thawed and combined. Fresh DTT was added to 10 mM. Prior to loading on a 320 ml S200 chromatography column, the protein sample was concentrated using an Amicon Ultra15 with a 10k MWCO. Final concentration was adjusted to 10 mgs/ml using SEC buffer(50 mM HEPES, pH 8.0, 3M GuHCl, 1 mM DTT (added fresh immediately prior to run)).

The column was equilibrated with 640 mls (2 CV's) of SEC Buffer at 1 ml/min (21°C). 50 mgs of denatured His₆-Parkin (5 mls @ 10 mgs/ml) starting material was loaded onto the column at 0.75 mls/min. Flow was increased to 1.5 mls/min 174 mls into the run. 5 ml fractions were collected and additional DTT was added to 10 mM final. Fractions were stored at 4°C until analyzed. When refolded as in Example 7, the resulting fraction ("#8BSEC") had activity about the same the "#8B" material.

### Example 10. Demonstration of Activity of Purified Parkin

An assay mixture containing His₆-Parkin prepared as described in Examples 6 and 7 was prepared. The 50 ul volume assay contained:
5 µM His₆-Parkin
100 nM human GST-E1 (Boston Biochem lot # 0271485,7.35 µM)
5 µM UbcH7 (Boston Biochem lot # 1070224)
100 µM Ubiquitin*
50 mM HEPES pH 7.5
50 mM NaCl
1 mM Mg-ATP (omitted from controls)
*29.2 µl of 1 mM methylated ubiquitin (U-502, Boston Biochem lot# 2880574, dissolved in dH₂O to 1 mM) was used to resuspend 50 µg of biotin-ubiquitin (UB-560, Boston Biochem lot # 2011584). This results in 30µl of 1.17 mM ubiquitin with 17% biotinylated.

The reaction was incubated at 37°C for 0,15,30,60 or 90 minutes; and reactions terminated by adding 6 µl 5X sample buffer (250 mM HEPES pH 7.5; 250 mM NaCl) plus 4 µl 1M DTT.

A 15 µl aliquot of the assay mixture was electrophoresed on an 12% polyacrylaminde gel and transferred to a polyvinylidene fluoride (PVDF) membrane overnight (25V in 10mM CAPS, pH 11, 10% MeOH, 4°C) for Western blotting. The membrane was blocked 2 hours in TBST + 5% BSA and incubated 1 hour at room temperature with NeutrAvidin-HRP (dilution: 1:7,500) in TBST + 3% BSA (1 hour at room temperature). The membrane was washed 8 X 15 minutes with room temperature TBST. Uniform transfer from gel to membrane was confirmed by Ponceau S staining.

The results are shown in Figure 7. Note that His₆-Parkin migrates at 57 kDa, UbcH7 at 18 kDa, Ub at 8.6 kDa, and His₆-Parkin-Ub complex at greater than 65 kDa. A distinct banding pattern was seen in the 66 kDa region starting at 15 minutes in reactions containing ATP. No ligase-dependent activity was observed in the absence of ATP at up to 90 minutes. Some signal was observed in the 66 kDa region when Parkin, E1, ubiquitin, and ATP were incubated for 90 minutes (lane 4).

The results shown in Figure 7 are consistent with other experiments demonstrating ligase activity of *E. coli* produced Parkin, beginning at 15 minutes reaction time. The activity appears to plateau by 60 minutes-a further increase in product was not seen at 90 minutes. Generation of the distinct doublet product in the 60kD region (as well as a high molecular weight smear) requires ATP; this eliminates the possibility that ubiquitin is simply co-aggregating with Parkin in a non-specific fashion. However, some reaction product was seen in the absence of UbcH7 when Parkin was incubated with E1 and ubiquitin for 90 minutes. This apparent ubiquitination of Parkin in the absence of UbcH7 is most likely a non-specific interaction between Parkin and E1. The amount of product generated at 90 minutes in this reaction was much less than the amount of product generated in the presence of UbcH7 at 15 minutes (compare lanes 4 and 12).

## Claims

1. An in vitro cell-based assay for identifying a candidate compound for treatment of Parkinson's Disease comprising
(a) exposing a mammalian cell expressing Parkin to a test agent;
(b) comparing proteasome function in the cell and proteasome function characteristic of a corresponding mammalian cell expressing Parkin not exposed to the test agent;
wherein an increased level of proteasome function in the cell exposed to the test agent indicates the agent is a candidate compound for treatment of Parkinson's Disease.

2. The cell-based assay of claim 1 wherein the mammalian cells express GFPu and proteasome function is measured by measuring the amount of GFPu in the cells.

3. The cell-based assay of claim 2 wherein the amount of GFPu in the cells is determined by measuring GFPu fluorescence.

4. The cell-based assay of claim 1 further comprising a proteasome function assay comprising
(i) exposing a mammalian cell expressing a mutant Parkin to the candidate compound; and
(ii) comparing proteasome function in the cell in (i) and proteasome function characteristic of a cell expressing the mutant Parkin and not exposed to the candidate compound.

5. The cell-based assay of claim 4, wherein the mutant Parkin is R42P, S167N, C212Y, T240M, R275W, C289G, or P437L Parkin.

6. The cell-based assay of claim 1, further comprising a proteasome function assay comprising (i) exposing a mammalian cell expressing Huntingtin to the candidate compound, and (ii) comparing proteasome function in the cell in (i) and proteasome function characteristic of a cell expressing Huntingtin not exposed to the candidate compound.

7. The cell-based assay of claim 1, further comprising an *in vitro* activity assay comprising (i) measuring the autoubiquitination activity of a purified Parkin protein in the presence of the compound, and (ii) comparing the autoubiquitination activity of purified Parkin protein in the presence of the compound with autoubiquitination activity of purified Parkin protein in the absence of the compound.

8. The cell-based assay of claim 1, further comprising an *in vitro* activity binding assay comprising (i) contacting the compound with purified Parkin protein, and (ii) detecting the binding, if any, of the compound and the Parkin protein

9. The cell-based assay of any one of claims 1 to 3, further comprising a proteasome function assay comprising:
(a) exposing a mammalian cell expressing a non-Parkin protein to the candidate compound; and
(b) comparing proteasome function in the cell with proteasome function characteristic of a cell expressing the non-Parkin protein not exposed to the candidate compound;
wherein an increase in proteasome function in cells expressing Parkin, but not in cells expressing the non-Parkin protein indicates the candidate compound specifically modulates the effect of Parkin on proteasomes.

10. The cell-based assay of claim 9, wherein the non-Parkin protein is SOD1, Rhodopsin, connexin 43, UB+1, or presenilin.

11. The cell-based assay of any one of claims 1-3, 6, 9 or 10, wherein the mammalian cell expresses recombinant wild-type Parkin.

12. The cell-based assay of any one of the preceding claims, wherein the test agent has a molecular weight less than 1000.

## Patentansprüche

1. In-vitro-Testverfahren auf Zellbasis zur Identifizierung einer Kandidatenverbindung zur Behandlung von Morbus Parkinson, bei dem man
(a)eine Parkin exprimierende Säugerzelle einem Testagens aussetzt;
(b)die Proteasomenfunktion in der Zelle und die für eine entsprechende, Parkin exprimierende Säugerzelle, die nicht dem Testagens ausgesetzt wurde, charakteristische Proteasomenfunktion vergleicht;
wobei ein erhöhtes Niveau der Proteasomenfunktion in der dem Testagens ausgesetzten Zelle anzeigt, dass es sich bei dem Agens um eine Kandidatenverbindung zur Behandlung von Morbus Parkinson handelt.

2. Testverfahren auf Zellbasis nach Anspruch 1, wobei die Säugerzellen GFPu exprimieren und die Proteasomenfunktion über die Messung der Menge an GFPu in den Zellen gemessen wird.

3. Testverfahren auf Zellbasis nach Anspruch 2, wobei die Menge an GFPu in den Zellen durch Messen von GFPu-Fluoreszenz bestimmt wird.

4. Testverfahren auf Zellbasis nach Anspruch 1, ferner umfassend einen Proteasomenfunktionstest, bei dem man
(i) eine eine Parkin-Mutante exprimierende Säugerzelle der Kandidatenverbindung aussetzt und
(ii) die Proteasomenfunktion in der Zelle in (i) und die für eine die Parkin-Mutante exprimierende Zelle, die nicht der Kandidatenverbindung ausgesetzt wurde, charakteristische Proteasomenfunktion vergleicht.

5. Testverfahren auf Zellbasis nach Anspruch 4, wobei es sich bei der Parkin-Mutante um R42P-, S167N-, C212Y-, T240M-, R275W-, C289G- oder P437L-Parkin handelt.

6. Testverfahren auf Zellbasis nach Anspruch 1, ferner umfassend einen Proteasomenfunktionstest, bei dem man (i) eine Huntingtin exprimierende Säugerzelle der Kandidatenverbindung aussetzt und (ii) die Proteasomenfunktion in der Zelle in (i) und die für eine Huntingtin exprimierende Zelle, die nicht der Kandidatenverbindung ausgesetzt wurde, charakteristische Proteasomenfunktion vergleicht.

7. Testverfahren auf Zellbasis nach Anspruch 1, ferner umfassend einen In-vitro-Aktivitätstest, bei dem man (i) die Autoubiquitinierungsaktivität eines aufgereinigten Parkin-Proteins in Gegenwart der Verbindung misst und (ii) die Autoubiquitinierungsaktivität des aufgereinigten Parkin-Proteins in Gegenwart der Verbindung mit der Autoubiquitinierungsaktivität des aufgereinigten Parkin-Proteins in Abwesenheit der Verbindung vergleicht.

8. Testverfahren auf Zellbasis nach Anspruch 1, ferner umfassend einen In-vitro-Aktivitätsbindungstest, bei dem man (i) die Verbindung mit aufgereinigtem Parkin-Protein in Kontakt bringt und (ii) eine eventuelle Bindung der Verbindung und des Parkin-Proteins nachweist.

9. Testverfahren auf Zellbasis nach einem der Ansprüche 1 bis 3, ferner umfassend einen Proteasomenfunktionstest, bei dem man
(a)eine ein Nicht-Parkin-Protein exprimierende Säugerzelle der Kandidatenverbindung aussetzt;
(b)die Proteasomenfunktion in der Zelle mit der für eine das Nicht-Parkin-Protein exprimierende Zelle, die nicht der Kandidatenverbindung ausgesetzt wurde, charakteristischen Proteasomenfunktion vergleicht;
wobei eine Erhöhung der Proteasomenfunktion in Parkin exprimierenden Zellen, aber nicht in Zellen, die das Nicht-Parkin-Protein exprimieren, anzeigt, dass die Kandidatenverbindung den Effekt von Parkin auf Proteasomen spezifisch moduliert.

10. Testverfahren auf Zellbasis nach Anspruch 9, wobei es sich bei dem Nicht-Parkin-Protein um SOD1, Rhodopsin, Connexin 43, UB+1 oder Präsenilin handelt.

11. Testverfahren auf Zellbasis nach einem der Ansprüche 1-3, 6, 9 oder 10, wobei die Säugerzelle rekombinantes Wildtyp-Parkin exprimiert.

12. Testverfahren auf Zellbasis nach einem der vorhergehenden Ansprüche, wobei das Testagens ein Molekulargewicht von weniger als 1000 aufweist.

## Revendications

1. Essai *in vitro* à base de cellules pour identifier un composé candidat pour le traitement de la maladie de Parkinson comprenant
(a) l'exposition d'une cellule mammalienne exprimant la parkine à un agent d'essai ;
b) la comparaison de la fonction de protéasome dans la cellule et de la fonction de protéasome caractéristique d'une cellule mammalienne correspondante exprimant la parkine non exposée à l'agent d'essai ;
dans lequel un taux augmenté de fonction de protéasome dans la cellule exposée à l'agent expérimental indique que l'agent est un composé candidat pour le traitement de la maladie de Parkinson.

2. Essai à base de cellules de la revendication 1 dans lequel les cellules mammaliennes expriment GFPu et la fonction de protéasome est mesurée en mesurant la quantité de GFPu dans les cellules.

3. Essai à base de cellules de la revendication 2 dans lequel la quantité de GFPu dans les cellules est déterminée en mesurant la fluorescence de GFPu.

4. Essai à base de cellules de la revendication 1 comprenant en outre un essai de la fonction de protéasome comprenant
(i) l'exposition d'une cellule mammalienne exprimant une parkine mutante au composé candidat ; et
(ii) la comparaison de la fonction de protéasome dans la cellule dans (i) et la fonction de protéasome caractéristique d'une cellule exprimant la parkine mutante et non exposée au composé candidat.

5. Essai à base de cellules de la revendication 4, dans lequel le mutant de parkine est la parkine R42P, S167N, C212Y, T240M, R275W, C289G, ou P437L.

6. Essai à base de cellules de la revendication 1, comprenant en outre un essai de fonction de protéasome comprenant (i) l'exposition d'une cellule mammalienne exprimant la huntingtine au composé candidat, et (ii) la comparaison de la fonction de protéasome dans la cellule dans (i) et la fonction de protéasome caractéristique d'une cellule exprimant la huntingtine non exposée au composé candidat.

7. Essai à base de cellules de la revendication 1, comprenant en outre une essai d'activité *in vitro* comprenant (i) la mesure de l'activité d'autoubiquitination d'une protéine parkine purifiée en présence du composé, et (ii) la comparaison de l'activité d'auto-ubiquitination de protéine parkine purifiée en présence du composé à l'activité d'autoubiquitination de protéine parkine purifiée en l'absence du composé.

8. Essai à base de cellules de la revendication 1, comprenant en outre un essai d'activité de liaison *in vitro* comprenant (i) la mise en contact du composé avec la protéine parkine purifiée, et (ii) la détection de la liaison, le cas échéant, du composé et de la protéine parkine.

9. Essai à base de cellules de l'une quelconque des revendications 1 à 3, comprenant en outre un essai de fonction de protéasome comprenant :
(a) l'exposition d'une cellule mammalienne exprimant une protéine non-parkine au composé candidat ; et
(b) la comparaison de la fonction de protéasome dans la cellule à la fonction de protéasome caractéristique d'une cellule exprimant la protéine non-parkine non exposée au composé candidat ;
où une augmentation de la fonction de protéasome dans des cellules exprimant la parkine mais pas dans les cellules exprimant la protéine non-parkine indique que le composé candidat module spécifiquement l'effet de la parkine sur les protéasomes.

10. Essai à base de cellules de la revendication 9, dans lequel la protéine non-parkine est SOD1, la rhodopsine, la connexine 43, UB+1, ou la préséniline.

11. Essai à base de cellules de l'une quelconque des revendications 1 à 3, 6, 9 ou 10, dans lequel la cellule mammalienne exprime une parkine de type sauvage recombinante.

12. Essai à base de cellules de l'une quelconque des revendications précédentes, dans lequel l'agent d'essai a un poids moléculaire inférieur à 1000.


### SEQUENCE LISTING

<110> Johnston, Jennifer A.
<120> ASSAY FOR PARKINSON'S DISEASE THERAPEUTICS AND ENZYMATICALLY ACTIVE PARKIN PREPARATIONS USEFUL THEREIN
<130> 015270-018110US
<140> US 00/000,000
   <141> 2006-12-12
<150> US 60/749,964
   <151> 2005-12-12
<160> 11
<170> PatentIn version 3.3
<210> 1
   <211> 1398
   <212> DNA
   <213> Homo sapiens
<400> 1
   atgatagtgt ttgtcaggtt caactccagc catggtttcc cagtggaggt cgattctgac      60
   accagcatct tccagctcaa ggaggtggtt gctaagcgac agggggttcc ggctgaccag      120
   ttgcgtgtga ttttcgcagg gaaggagctg aggaatgact ggactgtgca gaattgtgac     180
   ctggatcagc agagcattgt tcacattgtg cagagaccgt ggagaaaagg tcaagaaatg     240
   aatgcaactg gaggcgacga ccccagaaac gcggcgggag gctgtgagcg ggagccccag     300
   agcttgactc gggtggacct cagcagctca gtcctcccag gagactctgt ggggctggct     360
   gtcattctgc acactgacag caggaaggac tcaccaccag ctggaagtcc agcaggtaga     420
   tcaatctaca acagctttta tgtgtattgc aaaggcccct gtcaaagagt gcagccggga     480
   aaactcaggg tacagtgcag cacctgcagg caggcaacgc tcaccttgac ccagggtcca     540
   tcttgctggg atgatgtttt aattccaaac cggatgagtg gtgaatgcca atccccacac     600
   tgccctggga ctagtgcaga atttttcttt aaatgtggag cacaccccac ctctgacaag     660
   gaaacaccag tagctttgca cctgatcgca acaaatagtc ggaacatcac ttgcattacg     720
   tgcacagacg tcaggagccc cgtcctggtt ttccagtgca actcccgcca cgtgatttgc     780
   ttagactgtt tccacttata ctgtgtgaca agactcaatg atcggcagtt tgttcacgac     840
   cctcaacttg gctactccct gccttgtgtg gctggctgtc ccaactcctt gattaaagag     900
   ctccatcact tcaggattct gggagaagag cagtacaacc ggtaccagca gtatggtgca     960
   gaggagtgtg tcctgcagat ggggggcgtg ttatgccccc gccctggctg tggagcgggg     1020
   ctgctgccgg agcctgacca gaggaaagtc acctgcgaag ggggcaatgg cctgggctgt      1080
   gggtttgcct tctgccggga atgtaaagaa gcgtaccatg aaggggagtg cagtgccgta    1140
   tttgaagcct caggaacaac tactcaggcc tacagagtcg atgaaagagc cgccgagcag    1200
   gctcgttggg aagcagcctc caaagaaacc atcaagaaaa ccaccaagcc ctgtccccgc    1260
   tgccatgtac cagtggaaaa aaatggaggc tgcatgcaca tgaagtgtcc gcagccccag 1320
   tgcaggctcg agtggtgctg gaactgtggc tgcgagtgga accgcgtctg catgggggac 1380
   cactggttcg acgtgtag 1398
<210> 2
   <211> 465
   <212> PRT
   <213> Homo sapiens
<400> 2
   Met Ile Val Phe Val Arg Phe Asn Ser Ser His Gly Phe Pro Val Glu
   1               5                   10                  15
   Val Asp Ser Asp Thr Ser Ile Phe Gln Leu Lys Glu Val Val Ala Lys
               20                  25                  30
   Arg Gln Gly Val Pro Ala Asp Gln Leu Arg Val Ile Phe Ala Gly Lys
           35                  40                  45
   Glu Leu Arg Asn Asp Trp Thr Val Gln Asn Cys Asp Leu Asp Gln Gln
       50                  55                  60
   Ser Ile Val His Ile Val Gln Arg Pro Trp Arg Lys Gly Gln Glu Met
   65                  70                  75                  80
   Asn Ala Thr Gly Gly Asp Asp Pro Arg Asn Ala Ala Gly Gly Cys Glu
                   85                  90                  95
   Arg Glu Pro Gln Ser Leu Thr Arg Val Asp Leu Ser Ser Ser Val Leu
               100                 105                 110
   Pro Gly Asp Ser Val Gly Leu Ala Val Ile Leu His Thr Asp Ser Arg
           115                 120                 125
   Lys Asp Ser Pro Pro Ala Gly Ser Pro Ala Gly Arg Ser Ile Tyr Asn
       130                 135                 140
   Ser Phe Tyr Val Tyr Cys Lys Gly Pro Cys Gln Arg Val Gln Pro Gly
   145                 150                 155                 160
   Lys Leu Arg Val Gln Cys Ser Thr Cys Arg Gln Ala Thr Leu Thr Leu
                   165                 170                 175
   Thr Gln Gly Pro Ser Cys Trp Asp Asp Val Leu Ile Pro Asn Arg Met
               180                 185                 190
   Ser Gly Glu Cys Gln Ser Pro His Cys Pro Gly Thr Ser Ala Glu Phe
           195                 200                 205
   Phe Phe Lys Cys Gly Ala His Pro Thr Ser Asp Lys Glu Thr Pro Val
       210                 215                 220
   Ala Leu His Leu Ile Ala Thr Asn Ser Arg Asn Ile Thr Cys Ile Thr
   225                 230                 235                 240
   Cys Thr Asp Val Arg Ser Pro Val Leu Val Phe Gln Cys Asn Ser Arg
                   245                 250                 255
   His Val Ile Cys Leu Asp Cys Phe His Leu Tyr Cys Val Thr Arg Leu
               260                 265                 270
   Asn Asp Arg Gln Phe Val His Asp Pro Gln Leu Gly Tyr Ser Leu Pro
           275                 280                 285
   Cys Val Ala Gly Cys Pro Asn Ser Leu Ile Lys Glu Leu His His Phe
       290                 295                 300
   Arg Ile Leu Gly Glu Glu Gln Tyr Asn Arg Tyr Gln Gln Tyr Gly Ala
   305                 310                 315                 320
   Glu Glu Cys Val Leu Gln Met Gly Gly Val Leu Cys Pro Arg Pro Gly
                   325                 330                 335
   Cys Gly Ala Gly Leu Leu Pro Glu Pro Asp Gln Arg Lys Val Thr Cys
               340                 345                 350
   Glu Gly Gly Asn Gly Leu Gly Cys Gly Phe Ala Phe Cys Arg Glu Cys
           355                 360                 365
   Lys Glu Ala Tyr His Glu Gly Glu Cys Ser Ala Val Phe Glu Ala Ser
       370                 375                 380
   Gly Thr Thr Thr Gln Ala Tyr Arg Val Asp Glu Arg Ala Ala Glu Gln
   385                 390                 395                 400
   Ala Arg Trp Glu Ala Ala Ser Lys Glu Thr Ile Lys Lys Thr Thr Lys
                   405                 410                 415
   Pro Cys Pro Arg Cys His Val Pro Val Glu Lys Asn Gly Gly Cys Met
               420                 425                 430
   His Met Lys Cys Pro Gln Pro Gln Cys Arg Leu Glu Trp Cys Trp Asn
           435                 440                 445
   Cys Gly Cys Glu Trp Asn Arg Val Cys Met Gly Asp His Trp Phe Asp
       450                 455                 460
   Val
   465
<210> 3
   <211> 1395
   <212> DNA
   <213> Mus sp.
<400> 3
   atgatagtgt ttgtcaggtt caactccagc tatggcttcc cagtggaggt cgattctgac      60
   accagcatct tgcagctcaa ggaagtggtt gctaagcgac agggggttcc agctgaccag    120
   ctgcgtgtga tttttgccgg gaaggagctt ccgaatcacc tgacggttca aaactgtgac    180
   ctggaacaac agagtattgt acacatagta cagagaccac ggaggagaag tcatgaaaca    240
   aatgcatctg gaggggacga accccagagc acctcagagg gctccatatg ggagtccagg    300
   agcttgacac gagtggacct gagcagccat accctgccgg tggactctgt ggggctggcg    360
   gtcattctgg acacagacag taagagggat tcagaagcag ccagaggtcc agttaaaccc    420
   acctacaaca gctttttcat ctactgcaaa ggcccctgcc acaaggtcca gcctggaaag    480
   ctccgagttc agtgtggcac ctgcaaacaa gcaaccctca ccttggccca gggcccatct    540
   tgctgggacg atgtcttaat tccaaaccgg atgagtggtg agtgccagtc tccagactgc    600
   cctggaacca gagctgaatt tttctttaaa tgtggagcac acccaacctc agacaaggac    660
   acgtcggtag ctttgaacct gatcaccagc aacaggcgca gcatcccttg catagcgtgc    720
   acagatgtca ggagccctgt cctggtcttc cagtgtaacc accgtcacgt gatctgtttg    780
   gactgtttcc acttgtattg tgtcacaaga ctcaacgatc ggcagtttgt ccacgatgct    840
   caacttggct actccctgcc gtgtgtagct ggctgtccca actccctgat taaagagctc    900
   catcacttca ggatccttgg agaagagcag tacactaggt accagcagta tggggccgag    960
   gaatgcgtgc tgcaaatggg aggtgtgctg tgcccccgtc ctggctgtgg agctggactg   1020
   ctacctgaac agggccagag gaaagtcacc tgcgaagggg gcaacggcct gggctgcggg   1080
   tttgttttct gccgggactg taaggaagca taccatgaag gggattgcga ctcactgctc   1140
   gaaccctcag gagccacttc tcaggcctac agggtggaca aaagagccgc tgagcaagct   1200
   cgctgggagg aggcctccaa ggaaaccatc aagaagacca ccaagccttg tcctcgctgc   1260
   aacgtgccaa ttgaaaaaaa cggaggatgt atgcacatga agtgtcctca gccccagtgc   1320
   aagctggagt ggtgctggaa ctgtggctgt gagtggaacc gagcctgcat gggagatcac   1380
   tggtttgacg tgtag                                                    1395
<210> 4
   <211> 315
   <212> PRT
   <213> Mus sp.
<400> 4
   Met Val Val Arg Asn Ser Ser Tyr Gly Val Val Asp Ser Asp Thr Ser
   1               5                   10                  15
   Lys Val Val Ala Lys Arg Gly Val Ala Asp Arg Val Ala Gly Lys Asn
               20                  25                  30
   His Thr Val Asn Cys Asp Ser Val His Val Arg Arg Arg Arg Ser His
           35                  40                  45
   Thr Asn Ala Ser Gly Gly Asp Ser Thr Ser Gly Ser Trp Ser Arg Ser
       50                  55                  60
   Thr Arg Val Asp Ser Ser His Thr Val Asp Ser Val Gly Ala Val Asp
   65                  70                  75                  80
   Thr Asp Ser Lys Arg Asp Ser Ala Ala Arg Gly Val Lys Thr Tyr Asn
                   85                  90                  95
   Ser Tyr Cys Lys Gly Cys His Lys Val Gly Lys Arg Val Cys Gly Thr
               100                 105                 110
   Cys Lys Ala Thr Thr Ala Gly Ser Cys Trp Asp Asp Val Asn Arg Met
           115                 120                 125
   Ser Gly Cys Ser Asp Cys Gly Thr Arg Ala Lys Cys Gly Ala His Thr
       130                 135                 140
   Ser Asp Lys Asp Thr Ser Val Ala Asn Thr Ser Asn Arg Arg Ser Cys
   145                 150                 155                 160
   Ala Cys Thr Asp Val Arg Ser Val Val Cys Asn His Arg His Val Cys
                   165                 170                 175
   Asp Cys His Tyr Cys Val Thr Arg Asn Asp Arg Val His Asp Ala Gly
               180                 185                 190
   Tyr Ser Cys Val Ala Gly Cys Asn Ser Lys His His Arg Gly Tyr Thr
           195                 200                 205
   Arg Tyr Tyr Gly Ala Cys Val Met Gly Gly Val Cys Arg Gly Cys Gly
       210                 215                 220
   Ala Gly Gly Arg Lys Val Thr Cys Gly Gly Asn Gly Gly Cys Gly Val
   225                 230                 235                 240
   Cys Arg Asp Cys Lys Ala Tyr His Gly Asp Cys Asp Ser Ser Gly Ala
                   245                 250                 255
   Thr Ser Ala Tyr Arg Val Asp Lys Arg Ala Ala Ala Arg Trp Ala Ser
               260                 265                 270
   Lys Thr Lys Lys Thr Thr Lys Cys Arg Cys Asn Val Lys Asn Gly Gly
   275 280 285
   Cys Met His Met Lys Cys Cys Lys Trp Cys Trp Asn Cys Gly Cys Trp
   290 295 300
   Asn Arg Ala Cys Met Gly Asp His Trp Asp Val
   305 310 315
<210> 5
   <211> 1566
   <212> DNA
   <213> Artificial
<220>
   <223> Full length human Parkin fused to histidine tag
<220>
   <221> misc_feature
   <222> (14)..(29)
   <223> His sequence
<220>
   <221> misc_feature
   <222> (157)..(168)
   <223> Kozac sequence
<400> 5
   atatacatat gcaccatcat catcatcatt tcttctggtc tggtgccacg cggttctggt 60
   atgaaagaaa ccgctgctgc taaattcgaa cgccagcaca tggacagccc agatctgggt 120
   accgacgacg acgacaaggc catggctgat atcggatccg ccgccaccat gatagtgttt 180
   gtcaggttca actccagcca tggtttccca gtggaggtcg attctgacac cagcatcttc 240
   cagctcaagg aggtggttgc taagcgacag ggggttccgg ctgaccagtt gcgtgtgatt 300
   ttcgcaggga aggagctgag gaatgactgg actgtgcaga attgtgacct ggatcagcag 360
   agcattgttc acattgtgca gagaccgtgg agaaaaggtc aagaaatgaa tgcaactgga 420
   ggcgacgacc ccagaaacgc ggcgggaggc tgtgagcggg agccccagag cttgactcgg 480
   gtggacctca gcagctcagt cctcccagga gactctgtgg ggctggctgt cattctgcac 540
   actgacagca ggaaggactc accaccagct ggaagtccag caggtagatc aatctacaac 600
   agcttttatg tgtattgcaa aggcccctgt caaagagtgc agccgggaaa actcagggta 660
   cagtgcagca cctgcaggca ggcaacgctc accttgaccc agggtccatc ttgctgggat 720
   gatgttttaa ttccaaaccg gatgagtggt gaatgccaat ccccacactg ccctgggact 780
   agtgcagaat ttttctttaa atgtggagca caccccacct ctgacaagga aacaccagta 840
   gctttgcacc tgatcgcaac aaatagtcgg aacatcactt gcattacgtg cacagacgtc 900
   aggagccccg tcctggtttt ccagtgcaac tcccgccacg tgatttgctt agactgtttc 960
   cacttatact gtgtgacaag actcaatgat cggcagtttg ttcacgaccc tcaacttggc 1020
   tactccctgc cttgtgtggc tggctgtccc aactccttga ttaaagagct ccatcacttc 1080
   aggattctgg gagaagagca gtacaaccgg taccagcagt atggtgcaga ggagtgtgtc 1140
   ctgcagatgg ggggcgtgtt atgcccccgc cctggctgtg gagcggggct gctgccggag 1200
   cctgaccaga ggaaagtcac ctgcgaaggg ggcaatggcc tgggctgtgg gtttgccttc 1260
   tgccgggaat gtaaagaagc gtaccatgaa ggggagtgca gtgccgtatt tgaagcctca 1320
   ggaacaacta ctcaggccta cagagtcgat gaaagagccg ccgagcaggc tcgttgggaa 1380
   gcagcctcca aagaaaccat caagaaaacc accaagccct gtccccgctg ccatgtacca 1440
   gtggaaaaaa atggaggctg catgcacatg aagtgtccgc agccccagtg caggctcgag 1500
   tggtgctgga actgtggctg cgagtggaac cgcgtctgca tgggggacca ctggttcgac 1560
   gtgtag 1566
<210> 6
   <211> 720
   <212> DNA
   <213> Artificial
<220>
   <223> Green fluorescent protein
<400> 6
   atggtgagca agggcgagga gctgttcacc ggggtggtgc ccatcctggt cgagctggac      60
   ggcgacgtaa acggccacaa gttcagcgtg tccggcgagg gcgagggcga tgccacctac     120
   ggcaagctga ccctgaagtt catctgcacc accggcaagc tgcccgtgcc ctggcccacc     180
   ctcgtgacca ccctgaccta cggcgtgcag tgcttcagcc gctaccccga ccacatgaag     240
   cagcacgact tcttcaagtc cgccatgccc gaaggctacg tccaggagcg caccatcttc     300
   ttcaaggacg acggcaacta caagacccgc gccgaggtga agttcgaggg cgacaccctg     360
   gtgaaccgca tcgagctgaa gggcatcgac ttcaaggagg acggcaacat cctggggcac     420
   aagctggagt acaactacaa cagccacaac gtctatatca tggccgacaa gcagaagaac     480
   ggcatcaagg tgaacttcaa gatccgccac aacatcgagg acggcagcgt gcagctcgcc     540
   gaccactacc agcagaacac ccccatcggc gacggccccg tgctgctgcc cgacaaccac     600
   tacctgagca cccagtccgc cctgagcaaa gaccccaacg agaagcgcga tcacatggtc     660
   ctgctggagt tcgtgaccgc cgccgggatc actctcggca tggacgagct gtacaagtag     720
<210> 7
   <211> 239
   <212> PRT
   <213> Artificial
<220>
   <223> Green fluorescent protein
<400> 7
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 8
   <211> 60
   <212> DNA
   <213> Artificial
<220>
   <223> Degradation signal (Degron)
<400> 8
   gagatatcgc ttgcaaagaa ctggttctct tccttgagtc acttcgtaat tcacttgtag 60
<210> 9
   <211> 19
   <212> PRT
   <213> Artificial
<220>
   <223> Degradation signal (Degron)
<400> 9
   Glu Ile Ser Leu Ala Lys Asn Trp Phe Ser Ser Leu Ser His Phe Val
   1 5 10 15
   Ile His Leu
<210> 10
   <211> 1480
   <212> PRT
   <213> Artificial
<220>
   <223> CFTR protein
<400> 10
   Met Gln Arg Ser Pro Leu Glu Lys Ala Ser Val Val Ser Lys Leu Phe
   1               5                   10                  15
   Phe Ser Trp Thr Arg Pro Ile Leu Arg Lys Gly Tyr Arg Gln Arg Leu
               20                  25                  30
   Glu Leu Ser Asp Ile Tyr Gln Ile Pro Ser Val Asp Ser Ala Asp Asn
           35                  40                  45
   Leu Ser Glu Lys Leu Glu Arg Glu Trp Asp Arg Glu Leu Ala Ser Lys
       50                  55                  60
   Lys Asn Pro Lys Leu Ile Asn Ala Leu Arg Arg Cys Phe Phe Trp Arg
   65                  70                  75                  80
   Phe Met Phe Tyr Gly Ile Phe Leu Tyr Leu Gly Glu Val Thr Lys Ala
                   85                  90                  95
   Val Gln Pro Leu Leu Leu Gly Arg Ile Ile Ala Ser Tyr Asp Pro Asp
               100                 105                 110
   Asn Lys Glu Glu Arg Ser Ile Ala Ile Tyr Leu Gly Ile Gly Leu Cys
           115                 120                 125
   Leu Leu Phe Ile Val Arg Thr Leu Leu Leu His Pro Ala Ile Phe Gly
   130 135 140
   Leu His His Ile Gly Met Gln Met Arg Ile Ala Met Phe Ser Leu Ile
   145 150 155 160
   Tyr Lys Lys Thr Leu Lys Leu Ser Ser Arg Val Leu Asp Lys Ile Ser
   165 170 175
   Ile Gly Gln Leu Val Ser Leu Leu Ser Asn Asn Leu Asn Lys Phe Asp
   180 185 190
   Glu Gly Leu Ala Leu Ala His Phe Val Trp Ile Ala Pro Leu Gln Val
   195 200 205
   Ala Leu Leu Met Gly Leu Ile Trp Glu Leu Leu Gln Ala Ser Ala Phe
   210 215 220
   Cys Gly Leu Gly Phe Leu Ile Val Leu Ala Leu Phe Gln Ala Gly Leu
   225 230 235 240
   Gly Arg Met Met Met Lys Tyr Arg Asp Gln Arg Ala Gly Lys Ile Ser
   245 250 255
   Glu Arg Leu Val Ile Thr Ser Glu Met Ile Glu Asn Ile Gln Ser Val
   260 265 270
   Lys Ala Tyr Cys Trp Glu Glu Ala Met Glu Lys Met Ile Glu Asn Leu
   275 280 285
   Arg Gln Thr Glu Leu Lys Leu Thr Arg Lys Ala Ala Tyr Val Arg Tyr
   290 295 300
   Phe Asn Ser Ser Ala Phe Phe Phe Ser Gly Phe Phe Val Val Phe Leu
   305 310 315 320
   Ser Val Leu Pro Tyr Ala Leu Ile Lys Gly Ile Ile Leu Arg Lys Ile
   325 330 335
   Phe Thr Thr Ile Ser Phe Cys Ile Val Leu Arg Met Ala Val Thr Arg
   340 345 350
   Gln Phe Pro Trp Ala Val Gln Thr Trp Tyr Asp Ser Leu Gly Ala Ile
   355 360 365
   Asn Lys Ile Gln Asp Phe Leu Gln Lys Gln Glu Tyr Lys Thr Leu Glu
   370 375 380
   Tyr Asn Leu Thr Thr Thr Glu Val Val Met Glu Asn Val Thr Ala Phe
   385 390 395 400
   Trp Glu Glu Gly Phe Gly Glu Leu Phe Glu Lys Ala Lys Gln Asn Asn
   405 410 415
   Asn Asn Arg Lys Thr Ser Asn Gly Asp Asp Ser Leu Phe Phe Ser Asn
   420 425 430
   Phe Ser Leu Leu Gly Thr Pro Val Leu Lys Asp Ile Asn Phe Lys Ile
   435 440 445
   Glu Arg Gly Gln Leu Leu Ala Val Ala Gly Ser Thr Gly Ala Gly Lys
   450 455 460
   Thr Ser Leu Leu Met Met Ile Met Gly Glu Leu Glu Pro Ser Glu Gly
   465 470 475 480
   Lys Ile Lys His Ser Gly Arg Ile Ser Phe Cys Ser Gln Phe Ser Trp
   485 490 495
   Ile Met Pro Gly Thr Ile Lys Glu Asn Ile Ile Phe Gly Val Ser Tyr
   500 505 510
   Asp Glu Tyr Arg Tyr Arg Ser Val Ile Lys Ala Cys Gln Leu Glu Glu
   515 520 525
   Asp Ile Ser Lys Phe Ala Glu Lys Asp Asn Ile Val Leu Gly Glu Gly
   530 535 540
   Gly Ile Thr Leu Ser Gly Gly Gln Arg Ala Arg Ile Ser Leu Ala Arg
   545 550 555 560
   Ala Val Tyr Lys Asp Ala Asp Leu Tyr Leu Leu Asp Ser Pro Phe Gly
   565 570 575
   Tyr Leu Asp Val Leu Thr Glu Lys Glu Ile Phe Glu Ser Cys Val Cys
   580 585 590
   Lys Leu Met Ala Asn Lys Thr Arg Ile Leu Val Thr Ser Lys Met Glu
   595 600 605
   His Leu Lys Lys Ala Asp Lys Ile Leu Ile Leu Asn Glu Gly Ser Ser
   610 615 620
   Tyr Phe Tyr Gly Thr Phe Ser Glu Leu Gln Asn Leu Gln Pro Asp Phe
   625 630 635 640
   Ser Ser Lys Leu Met Gly Cys Asp Ser Phe Asp Gln Phe Ser Ala Glu
   645 650 655
   Arg Arg Asn Ser Ile Leu Thr Glu Thr Leu His Arg Phe Ser Leu Glu
   660 665 670
   Gly Asp Ala Pro Val Ser Trp Thr Glu Thr Lys Lys Gln Ser Phe Lys
   675 680 685
   Gln Thr Gly Glu Phe Gly Glu Lys Arg Lys Asn Ser Ile Leu Asn Pro
   690 695 700
   Ile Asn Ser Ile Arg Lys Phe Ser Ile Val Gln Lys Thr Pro Leu Gln
   705 710 715 720
   Met Asn Gly Ile Glu Glu Asp Ser Asp Glu Pro Leu Glu Arg Arg Leu
   725 730 735
   Ser Leu Val Pro Asp Ser Glu Gln Gly Glu Ala Ile Leu Pro Arg Ile
   740 745 750
   Ser Val Ile Ser Thr Gly Pro Thr Leu Gln Ala Arg Arg Arg Gln Ser
   755 760 765
   Val Leu Asn Leu Met Thr His Ser Val Asn Gln Gly Gln Asn Ile His
   770 775 780
   Arg Lys Thr Thr Ala Ser Thr Arg Lys Val Ser Leu Ala Pro Gln Ala
   785 790 795 800
   Asn Leu Thr Glu Leu Asp Ile Tyr Ser Arg Arg Leu Ser Gln Glu Thr
   805 810 815
   Gly Leu Glu Ile Ser Glu Glu Ile Asn Glu Glu Asp Leu Lys Glu Cys
   820 825 830
   Leu Phe Asp Asp Met Glu Ser Ile Pro Ala Val Thr Thr Trp Asn Thr
   835 840 845
   Tyr Leu Arg Tyr Ile Thr Val His Lys Ser Leu Ile Phe Val Leu Ile
   850 855 860
   Trp Cys Leu Val Ile Phe Leu Ala Glu Val Ala Ala Ser Leu Val Val
   865 870 875 880
   Leu Trp Leu Leu Gly Asn Thr Pro Leu Gln Asp Lys Gly Asn Ser Thr
   885 890 895
   His Ser Arg Asn Asn Ser Tyr Ala Val Ile Ile Thr Ser Thr Ser Ser
   900 905 910
   Tyr Tyr Val Phe Tyr Ile Tyr Val Gly Val Ala Asp Thr Leu Leu Ala
   915 920 925
   Met Gly Phe Phe Arg Gly Leu Pro Leu Val His Thr Leu Ile Thr Val
   930 935 940
   Ser Lys Ile Leu His His Lys Met Leu His Ser Val Leu Gln Ala Pro
   945 950 955 960
   Met Ser Thr Leu Asn Thr Leu Lys Ala Gly Gly Ile Leu Asn Arg Phe
   965 970 975
   Ser Lys Asp Ile Ala Ile Leu Asp Asp Leu Leu Pro Leu Thr Ile Phe
   980 985 990
   Asp Phe Ile Gln Leu Leu Leu Ile Val Ile Gly Ala Ile Ala Val Val
   995 1000 1005
   Ala Val Leu Gln Pro Tyr Ile Phe Val Ala Thr Val Pro Val Ile
   1010 1015 1020
   Val Ala Phe Ile Met Leu Arg Ala Tyr Phe Leu Gln Thr Ser Gln
   1025 1030 1035
   Gln Leu Lys Gln Leu Glu Ser Glu Gly Arg Ser Pro Ile Phe Thr
   1040 1045 1050
   His Leu Val Thr Ser Leu Lys Gly Leu Trp Thr Leu Arg Ala Phe
   1055 1060 1065
   Gly Arg Gln Pro Tyr Phe Glu Thr Leu Phe His Lys Ala Leu Asn
   1070 1075 1080
   Leu His Thr Ala Asn Trp Phe Leu Tyr Leu Ser Thr Leu Arg Trp
   1085 1090 1095
   Phe Gln Met Arg Ile Glu Met Ile Phe Val Ile Phe Phe Ile Ala
   1100 1105 1110
   Val Thr Phe Ile Ser Ile Leu Thr Thr Gly Glu Gly Glu Gly Arg
   1115 1120 1125
   Val Gly Ile Ile Leu Thr Leu Ala Met Asn Ile Met Ser Thr Leu
   1130 1135 1140
   Gln Trp Ala Val Asn Ser Ser Ile Asp Val Asp Ser Leu Met Arg
   1145 1150 1155
   Ser Val Ser Arg Val Phe Lys Phe Ile Asp Met Pro Thr Glu Gly
   1160 1165 1170
   Lys Pro Thr Lys Ser Thr Lys Pro Tyr Lys Asn Gly Gln Leu Ser
   1175 1180 1185
   Lys Val Met Ile Ile Glu Asn Ser His Val Lys Lys Asp Asp Ile
   1190 1195 1200
   Trp Pro Ser Gly Gly Gln Met Thr Val Lys Asp Leu Thr Ala Lys
   1205 1210 1215
   Tyr Thr Glu Gly Gly Asn Ala Ile Leu Glu Asn Ile Ser Phe Ser
   1220 1225 1230
   Ile Ser Pro Gly Gln Arg Val Gly Leu Leu Gly Arg Thr Gly Ser
   1235 1240 1245
   Gly Lys Ser Thr Leu Leu Ser Ala Phe Leu Arg Leu Leu Asn Thr
   1250 1255 1260
   Glu Gly Glu Ile Gln Ile Asp Gly Val Ser Trp Asp Ser Ile Thr
   1265 1270 1275
   Leu Gln Gln Trp Arg Lys Ala Phe Gly Val Ile Pro Gln Lys Val
   1280 1285 1290
   Phe Ile Phe Ser Gly Thr Phe Arg Lys Asn Leu Asp Pro Tyr Glu
   1295 1300 1305
   Gln Trp Ser Asp Gln Glu Ile Trp Lys Val Ala Asp Glu Val Gly
   1310 1315 1320
   Leu Arg Ser Val Ile Glu Gln Phe Pro Gly Lys Leu Asp Phe Val
   1325 1330 1335
   Leu Val Asp Gly Gly Cys Val Leu Ser His Gly His Lys Gln Leu
   1340 1345 1350
   Met Cys Leu Ala Arg Ser Val Leu Ser Lys Ala Lys Ile Leu Leu
   1355 1360 1365
   Leu Asp Glu Pro Ser Ala His Leu Asp Pro Val Thr Tyr Gln Ile
   1370 1375 1380
   Ile Arg Arg Thr Leu Lys Gln Ala Phe Ala Asp Cys Thr Val Ile
   1385 1390 1395
   Leu Cys Glu His Arg Ile Glu Ala Met Leu Glu Cys Gln Gln Phe
   1400 1405 1410
   Leu Val Ile Glu Glu Asn Lys Val Arg Gln Tyr Asp Ser Ile Gln
   1415 1420 1425
   Lys Leu Leu Asn Glu Arg Ser Leu Phe Arg Gln Ala Ile Ser Pro
   1430 1435 1440
   Ser Asp Arg Val Lys Leu Phe Pro His Arg Asn Ser Ser Lys Cys
   1445 1450 1455
   Lys Ser Lys Pro Gln Ile Ala Ala Leu Lys Glu Glu Thr Glu Glu
   1460 1465 1470
   Glu Val Gln Asp Thr Arg Leu
   1475 1480
<210> 11
   <211> 3142
   <212> PRT
   <213> Artificial
<220>
   <223> Huntingtin protein
<400> 11
   Met Ala Thr Leu Glu Lys Leu Met Lys Ala Phe Glu Ser Leu Lys Ser
   1 5 10 15
   Phe Gln Gln Gln Gln Gln Gln Gln Gln Gln Gln Gln Gln Gln Gln Gln
   20 25 30
   Gln Gln Gln Gln Gln Gln Pro Pro Pro Pro Pro Pro Pro Pro Pro Pro
   35 40 45
   Pro Gln Leu Pro Gln Pro Pro Pro Gln Ala Gln Pro Leu Leu Pro Gln
   50 55 60
   Pro Gln Pro Pro Pro Pro Pro Pro Pro Pro Pro Pro Gly Pro Ala Val
   65 70 75 80
   Ala Glu Glu Pro Leu His Arg Pro Lys Lys Glu Leu Ser Ala Thr Lys
   85 90 95
   Lys Asp Arg Val Asn His Cys Leu Thr Ile Cys Glu Asn Ile Val Ala
   100 105 110
   Gln Ser Val Arg Asn Ser Pro Glu Phe Gln Lys Leu Leu Gly Ile Ala
   115 120 125
   Met Glu Leu Phe Leu Leu Cys Ser Asp Asp Ala Glu Ser Asp Val Arg
   130 135 140
   Met Val Ala Asp Glu Cys Leu Asn Lys Val Ile Lys Ala Leu Met Asp
   145 150 155 160
   Ser Asn Leu Pro Arg Leu Gln Leu Glu Leu Tyr Lys Glu Ile Lys Lys
   165 170 175
   Asn Gly Ala Pro Arg Ser Leu Arg Ala Ala Leu Trp Arg Phe Ala Glu
   180 185 190
   Leu Ala His Leu Val Arg Pro Gln Lys Cys Arg Pro Tyr Leu Val Asn
   195 200 205
   Leu Leu Pro Cys Leu Thr Arg Thr Ser Lys Arg Pro Glu Glu Ser Val
   210 215 220
   Gln Glu Thr Leu Ala Ala Ala Val Pro Lys Ile Met Ala Ser Phe Gly
   225 230 235 240
   Asn Phe Ala Asn Asp Asn Glu Ile Lys Val Leu Leu Lys Ala Phe Ile
   245 250 255
   Ala Asn Leu Lys Ser Ser Ser Pro Thr Ile Arg Arg Thr Ala Ala Gly
   260 265 270
   Ser Ala Val Ser Ile Cys Gln His Ser Arg Arg Thr Gln Tyr Phe Tyr
   275 280 285
   Ser Trp Leu Leu Asn Val Leu Leu Gly Leu Leu Val Pro Val Glu Asp
   290 295 300
   Glu His Ser Thr Leu Leu Ile Leu Gly Val Leu Leu Thr Leu Arg Tyr
   305 310 315 320
   Leu Val Pro Leu Leu Gln Gln Gln Val Lys Asp Thr Ser Leu Lys Gly
   325 330 335
   Ser Phe Gly Val Thr Arg Lys Glu Met Glu Val Ser Pro Ser Ala Glu
   340 345 350
   Gln Leu Val Gln Val Tyr Glu Leu Thr Leu His His Thr Gln His Gln
   355 360 365
   Asp His Asn Val Val Thr Gly Ala Leu Glu Leu Leu Gln Gln Leu Phe
   370 375 380
   Arg Thr Pro Pro Pro Glu Leu Leu Gln Thr Leu Thr Ala Val Gly Gly
   385 390 395 400
   Ile Gly Gln Leu Thr Ala Ala Lys Glu Glu Ser Gly Gly Arg Ser Arg
   405 410 415
   Ser Gly Ser Ile Val Glu Leu Ile Ala Gly Gly Gly Ser Ser Cys Ser
   420 425 430
   Pro Val Leu Ser Arg Lys Gln Lys Gly Lys Val Leu Leu Gly Glu Glu
   435 440 445
   Glu Ala Leu Glu Asp Asp Ser Glu Ser Arg Ser Asp Val Ser Ser Ser
   450 455 460
   Ala Leu Thr Ala Ser Val Lys Asp Glu Ile Ser Gly Glu Leu Ala Ala
   465 470 475 480
   Ser Ser Gly Val Ser Thr Pro Gly Ser Ala Gly His Asp Ile Ile Thr
   485 490 495
   Glu Gln Pro Arg Ser Gln His Thr Leu Gln Ala Asp Ser Val Asp Leu
   500 505 510
   Ala Ser Cys Asp Leu Thr Ser Ser Ala Thr Asp Gly Asp Glu Glu Asp
   515 520 525
   Ile Leu Ser His Ser Ser Ser Gln Val Ser Ala Val Pro Ser Asp Pro
   530 535 540
   Ala Met Asp Leu Asn Asp Gly Thr Gln Ala Ser Ser Pro Ile Ser Asp
   545 550 555 560
   Ser Ser Gln Thr Thr Thr Glu Gly Pro Asp Ser Ala Val Thr Pro Ser
   565 570 575
   Asp Ser Ser Glu Ile Val Leu Asp Gly Thr Asp Asn Gln Tyr Leu Gly
   580 585 590
   Leu Gln Ile Gly Gln Pro Gln Asp Glu Asp Glu Glu Ala Thr Gly Ile
   595 600 605
   Leu Pro Asp Glu Ala Ser Glu Ala Phe Arg Asn Ser Ser Met Ala Leu
   610 615 620
   Gln Gln Ala His Leu Leu Lys Asn Met Ser His Cys Arg Gln Pro Ser
   625 630 635 640
   Asp Ser Ser Val Asp Lys Phe Val Leu Arg Asp Glu Ala Thr Glu Pro
   645 650 655
   Gly Asp Gln Glu Asn Lys Pro Cys Arg Ile Lys Gly Asp Ile Gly Gln
   660 665 670
   Ser Thr Asp Asp Asp Ser Ala Pro Leu Val His Cys Val Arg Leu Leu
   675 680 685
   Ser Ala Ser Phe Leu Leu Thr Gly Gly Lys Asn Val Leu Val Pro Asp
   690 695 700
   Arg Asp Val Arg Val Ser Val Lys Ala Leu Ala Leu Ser Cys Val Gly
   705 710 715 720
   Ala Ala Val Ala Leu His Pro Glu Ser Phe Phe Ser Lys Leu Tyr Lys
   725 730 735
   Val Pro Leu Asp Thr Thr Glu Tyr Pro Glu Glu Gln Tyr Val Ser Asp
   740 745 750
   Ile Leu Asn Tyr Ile Asp His Gly Asp Pro Gln Val Arg Gly Ala Thr
   755 760 765
   Ala Ile Leu Cys Gly Thr Leu Ile Cys Ser Ile Leu Ser Arg Ser Arg
   770 775 780
   Phe His Val Gly Asp Trp Met Gly Thr Ile Arg Thr Leu Thr Gly Asn
   785 790 795 800
   Thr Phe Ser Leu Ala Asp Cys Ile Pro Leu Leu Arg Lys Thr Leu Lys
   805 810 815
   Asp Glu Ser Ser Val Thr Cys Lys Leu Ala Cys Thr Ala Val Arg Asn
   820 825 830
   Cys Val Met Ser Leu Cys Ser Ser Ser Tyr Ser Glu Leu Gly Leu Gln
   835 840 845
   Leu Ile Ile Asp Val Leu Thr Leu Arg Asn Ser Ser Tyr Trp Leu Val
   850 855 860
   Arg Thr Glu Leu Leu Glu Thr Leu Ala Glu Ile Asp Phe Arg Leu Val
   865 870 875 880
   Ser Phe Leu Glu Ala Lys Ala Glu Asn Leu His Arg Gly Ala His His
   885 890 895
   Tyr Thr Gly Leu Leu Lys Leu Gln Glu Arg Val Leu Asn Asn Val Val
   900 905 910
   Ile His Leu Leu Gly Asp Glu Asp Pro Arg Val Arg His Val Ala Ala
   915 920 925
   Ala Ser Leu Ile Arg Leu Val Pro Lys Leu Phe Tyr Lys Cys Asp Gln
       930                 935                 940
   Gly Gln Ala Asp Pro Val Val Ala Val Ala Arg Asp Gln Ser Ser Val
   945                 950                 955                 960
   Tyr Leu Lys Leu Leu Met His Glu Thr Gln Pro Pro Ser His Phe Ser
                   965                 970                 975
   Val Ser Thr Ile Thr Arg Ile Tyr Arg Gly Tyr Asn Leu Leu Pro Ser
               980                 985                 990
   Ile Thr Asp Val Thr Met Glu Asn  Asn Leu Ser Arg Val  Ile Ala Ala
           995                 1000                1005
   Val Ser  His Glu Leu Ile Thr  Ser Thr Thr Arg Ala  Leu Thr Phe
       1010                1015                1020
   Gly Cys  Cys Glu Ala Leu Cys  Leu Leu Ser Thr Ala  Phe Pro Val
       1025                1030                1035
   Cys Ile  Trp Ser Leu Gly Trp  His Cys Gly Val Pro  Pro Leu Ser
       1040                1045                1050
   Ala Ser  Asp Glu Ser Arg Lys  Ser Cys Thr Val Gly  Met Ala Thr
       1055                1060                1065
   Met Ile  Leu Thr Leu Leu Ser  Ser Ala Trp Phe Pro  Leu Asp Leu
       1070                1075                1080
   Ser Ala  His Gln Asp Ala Leu  Ile Leu Ala Gly Asn  Leu Leu Ala
       1085                1090                1095
   Ala Ser  Ala Pro Lys Ser Leu  Arg Ser Ser Trp Ala  Ser Glu Glu
       1100                1105                1110
   Glu Ala  Asn Pro Ala Ala Thr  Lys Gln Glu Glu Val  Trp Pro Ala
       1115                1120                1125
   Leu Gly  Asp Arg Ala Leu Val  Pro Met Val Glu Gln  Leu Phe Ser
       1130                1135                1140
   His Leu  Leu Lys Val Ile Asn  Ile Cys Ala His Val  Leu Asp Asp
       1145                1150                1155
   Val Ala  Pro Gly Pro Ala Ile  Lys Ala Ala Leu Pro  Ser Leu Thr
       1160                1165                1170
   Asn Pro Pro Ser Leu Ser Pro Ile Arg Arg Lys Gly Lys Glu Lys
   1175 1180 1185
   Glu Pro Gly Glu Gln Ala Ser Val Pro Leu Ser Pro Lys Lys Gly
   1190 1195 1200
   Ser Glu Ala Ser Ala Ala Ser Arg Gln Ser Asp Thr Ser Gly Pro
   1205 1210 1215
   Val Thr Thr Ser Lys Ser Ser Ser Leu Gly Ser Phe Tyr His Leu
   1220 1225 1230
   Pro Ser Tyr Leu Lys Leu His Asp Val Leu Lys Ala Thr His Ala
   1235 1240 1245
   Asn Tyr Lys Val Thr Leu Asp Leu Gln Asn Ser Thr Glu Lys Phe
   1250 1255 1260
   Gly Gly Phe Leu Arg Ser Ala Leu Asp Val Leu Ser Gln Ile Leu
   1265 1270 1275
   Glu Leu Ala Thr Leu Gln Asp Ile Gly Lys Cys Val Glu Glu Ile
   1280 1285 1290
   Leu Gly Tyr Leu Lys Ser Cys Phe Ser Arg Glu Pro Met Met Ala
   1295 1300 1305
   Thr Val Cys Val Gln Gln Leu Leu Lys Thr Leu Phe Gly Thr Asn
   1310 1315 1320
   Leu Ala Ser Gln Phe Asp Gly Leu Ser Ser Asn Pro Ser Lys Ser
   1325 1330 1335
   Gln Gly Arg Ala Gln Arg Leu Gly Ser Ser Ser Val Arg Pro Gly
   1340 1345 1350
   Leu Tyr His Tyr Cys Phe Met Ala Pro Tyr Thr His Phe Thr Gln
   1355 1360 1365
   Ala Leu Ala Asp Ala Ser Leu Arg Asn Met Val Gln Ala Glu Gln
   1370 1375 1380
   Glu Asn Asp Thr Ser Gly Trp Phe Asp Val Leu Gln Lys Val Ser
   1385 1390 1395
   Thr Gln Leu Lys Thr Asn Leu Thr Ser Val Thr Lys Asn Arg Ala
   1400 1405 1410
   Asp Lys Asn Ala Ile His Asn His Ile Arg Leu Phe Glu Pro Leu
   1415 1420 1425
   Val Ile Lys Ala Leu Lys Gln Tyr Thr Thr Thr Thr Cys Val Gln
   1430 1435 1440
   Leu Gln Lys Gln Val Leu Asp Leu Leu Ala Gln Leu Val Gln Leu
   1445 1450 1455
   Arg Val Asn Tyr Cys Leu Leu Asp Ser Asp Gln Val Phe Ile Gly
   1460 1465 1470
   Phe Val Leu Lys Gln Phe Glu Tyr Ile Glu Val Gly Gln Phe Arg
   1475 1480 1485
   Glu Ser Glu Ala Ile Ile Pro Asn Ile Phe Phe Phe Leu Val Leu
   1490 1495 1500
   Leu Ser Tyr Glu Arg Tyr His Ser Lys Gln Ile Ile Gly Ile Pro
   1505 1510 1515
   Lys Ile Ile Gln Leu Cys Asp Gly Ile Met Ala Ser Gly Arg Lys
   1520 1525 1530
   Ala Val Thr His Ala Ile Pro Ala Leu Gln Pro Ile Val His Asp
   1535 1540 1545
   Leu Phe Val Leu Arg Gly Thr Asn Lys Ala Asp Ala Gly Lys Glu
   1550 1555 1560
   Leu Glu Thr Gln Lys Glu Val Val Val Ser Met Leu Leu Arg Leu
   1565 1570 1575
   Ile Gln Tyr His Gln Val Leu Glu Met Phe Ile Leu Val Leu Gln
   1580 1585 1590
   Gln Cys His Lys Glu Asn Glu Asp Lys Trp Lys Arg Leu Ser Arg
   1595 1600 1605
   Gln Ile Ala Asp Ile Ile Leu Pro Met Leu Ala Lys Gln Gln Met
   1610 1615 1620
   His Ile Asp Ser His Glu Ala Leu Gly Val Leu Asn Thr Leu Phe
   1625 1630 1635
   Glu Ile Leu Ala Pro Ser Ser Leu Arg Pro Val Asp Met Leu Leu
   1640 1645 1650
   Arg Ser Met Phe Val Thr Pro Asn Thr Met Ala Ser Val Ser Thr
   1655 1660 1665
   Val Gln Leu Trp Ile Ser Gly Ile Leu Ala Ile Leu Arg Val Leu
       1670                1675                1680
   Ile Ser Gln Ser Thr Glu Asp Ile Val Leu Ser Arg Ile Gln Glu
       1685                1690                1695
   Leu Ser Phe Ser Pro Tyr Leu Ile Ser Cys Thr Val Ile Asn Arg
       1700                1705                1710
   Leu Arg Asp Gly Asp Ser Thr Ser Thr Leu Glu Glu His Ser Glu
       1715                1720                1725
   Gly Lys Gln Ile Lys Asn Leu Pro Glu Glu Thr Phe Ser Arg Phe
       1730                1735                1740
   Leu Leu Gln Leu Val Gly Ile Leu Leu Glu Asp Ile Val Thr Lys
       1745                1750                1755
   Gln Leu Lys Val Glu Met Ser Glu Gln Gln His Thr Phe Tyr Cys
       1760                1765                1770
   Gln Glu Leu Gly Thr Leu Leu Met Cys Leu Ile His Ile Phe Lys
       1775                1780                1785
   Ser Gly Met Phe Arg Arg Ile Thr Ala Ala Ala Thr Arg Leu Phe
       1790                1795                1800
   Arg Ser Asp Gly Cys Gly Gly Ser Phe Tyr Thr Leu Asp Ser Leu
       1805                1810                1815
   Asn Leu Arg Ala Arg Ser Met Ile Thr Thr His Pro Ala Leu Val
       1820                1825                1830
   Leu Leu Trp Cys Gln Ile Leu Leu Leu Val Asn His Thr Asp Tyr
       1835                1840                1845
   Arg Trp Trp Ala Glu Val Gln Gln Thr Pro Lys Arg His Ser Leu
       1850                1855                1860
   Ser Ser Thr Lys Leu Leu Ser Pro Gln Met Ser Gly Glu Glu Glu
       1865                1870                1875
   Asp Ser Asp Leu Ala Ala Lys Leu Gly Met Cys Asn Arg Glu Ile
       1880                1885                1890
   Val Arg Arg Gly Ala Leu Ile Leu Phe Cys Asp Tyr Val Cys Gln
       1895                1900                1905
   Asn Leu His Asp Ser Glu His Leu Thr Trp Leu Ile Val Asn His
       1910                1915                1920
   Ile Gln  Asp Leu Ile Ser Leu  Ser His Glu Pro Pro  Val Gln Asp
       1925                1930                1935
   Phe Ile  Ser Ala Val His Arg  Asn Ser Ala Ala Ser  Gly Leu Phe
       1940                1945                1950
   Ile Gln  Ala Ile Gln Ser Arg  Cys Glu Asn Leu Ser  Thr Pro Thr
       1955                1960                1965
   Met Leu  Lys Lys Thr Leu Gln  Cys Leu Glu Gly Ile  His Leu Ser
       1970                1975                1980
   Gln Ser  Gly Ala Val Leu Thr  Leu Tyr Val Asp Arg  Leu Leu Cys
       1985                1990                1995
   Thr Pro  Phe Arg Val Leu Ala  Arg Met Val Asp Ile  Leu Ala Cys
       2000                2005                2010
   Arg Arg  Val Glu Met Leu Leu  Ala Ala Asn Leu Gln  Ser Ser Met
       2015                2020                2025
   Ala Gln  Leu Pro Met Glu Glu  Leu Asn Arg Ile Gln  Glu Tyr Leu
       2030                2035                2040
   Gln Ser  Ser Gly Leu Ala Gln  Arg His Gln Arg Leu  Tyr Ser Leu
       2045                2050                2055
   Leu Asp  Arg Phe Arg Leu Ser  Thr Met Gln Asp Ser  Leu Ser Pro
       2060                2065                2070
   Ser Pro  Pro Val Ser Ser His  Pro Leu Asp Gly Asp  Gly His Val
       2075                2080                2085
   Ser Leu  Glu Thr Val Ser Pro  Asp Lys Asp Trp Tyr  Val His Leu
       2090                2095                2100
   Val Lys  Ser Gln Cys Trp Thr  Arg Ser Asp Ser Ala  Leu Leu Glu
       2105                2110                2115
   Gly Ala  Glu Leu Val Asn Arg  Ile Pro Ala Glu Asp  Met Asn Ala
       2120                2125                2130
   Phe Met  Met Asn Ser Glu Phe  Asn Leu Ser Leu Leu  Ala Pro Cys
       2135                2140                2145
   Leu Ser  Leu Gly Met Ser Glu  Ile Ser Gly Gly Gln  Lys Ser Ala
       2150                2155                2160
   Leu Phe Glu Ala Ala Arg Glu Val Thr Leu Ala Arg Val Ser Gly
   2165 2170 2175
   Thr Val Gln Gln Leu Pro Ala Val His His Val Phe Gln Pro Glu
   2180 2185 2190
   Leu Pro Ala Glu Pro Ala Ala Tyr Trp Ser Lys Leu Asn Asp Leu
   2195 2200 2205
   Phe Gly Asp Ala Ala Leu Tyr Gln Ser Leu Pro Thr Leu Ala Arg
   2210 2215 2220
   Ala Leu Ala Gln Tyr Leu Val Val Val Ser Lys Leu Pro Ser His
   2225 2230 2235
   Leu His Leu Pro Pro Glu Lys Glu Lys Asp Ile Val Lys Phe Val
   2240 2245 2250
   Val Ala Thr Leu Glu Ala Leu Ser Trp His Leu Ile His Glu Gln
   2255 2260 2265
   Ile Pro Leu Ser Leu Asp Leu Gln Ala Gly Leu Asp Cys Cys Cys
   2270 2275 2280
   Leu Ala Leu Gln Leu Pro Gly Leu Trp Ser Val Val Ser Ser Thr
   2285 2290 2295
   Glu Phe Val Thr His Ala Cys Ser Leu Ile Tyr Cys Val His Phe
   2300 2305 2310
   Ile Leu Glu Ala Val Ala Val Gln Pro Gly Glu Gln Leu Leu Ser
   2315 2320 2325
   Pro Glu Arg Arg Thr Asn Thr Pro Lys Ala Ile Ser Glu Glu Glu
   2330 2335 2340
   Glu Glu Val Asp Pro Asn Thr Gln Asn Pro Lys Tyr Ile Thr Ala
   2345 2350 2355
   Ala Cys Glu Met Val Ala Glu Met Val Glu Ser Leu Gln Ser Val
   2360 2365 2370
   Leu Ala Leu Gly His Lys Arg Asn Ser Gly Val Pro Ala Phe Leu
   2375 2380 2385
   Thr Pro Leu Leu Arg Asn Ile Ile Ile Ser Leu Ala Arg Leu Pro
   2390 2395 2400
   Leu Val Asn Ser Tyr Thr Arg Val Pro Pro Leu Val Trp Lys Leu
   2405 2410 2415
   Gly Trp Ser Pro Lys Pro Gly Gly Asp Phe Gly Thr Ala Phe Pro
   2420 2425 2430
   Glu Ile Pro Val Glu Phe Leu Gln Glu Lys Glu Val Phe Lys Glu
   2435 2440 2445
   Phe Ile Tyr Arg Ile Asn Thr Leu Gly Trp Thr Ser Arg Thr Gln
   2450 2455 2460
   Phe Glu Glu Thr Trp Ala Thr Leu Leu Gly Val Leu Val Thr Gln
   2465 2470 2475
   Pro Leu Val Met Glu Gln Glu Glu Ser Pro Pro Glu Glu Asp Thr
   2480 2485 2490
   Glu Arg Thr Gln Ile Asn Val Leu Ala Val Gln Ala Ile Thr Ser
   2495 2500 2505
   Leu Val Leu Ser Ala Met Thr Val Pro Val Ala Gly Asn Pro Ala
   2510 2515 2520
   Val Ser Cys Leu Glu Gln Gln Pro Arg Asn Lys Pro Leu Lys Ala
   2525 2530 2535
   Leu Asp Thr Arg Phe Gly Arg Lys Leu Ser Ile Ile Arg Gly Ile
   2540 2545 2550
   Val Glu Gln Glu Ile Gln Ala Met Val Ser Lys Arg Glu Asn Ile
   2555 2560 2565
   Ala Thr His His Leu Tyr Gln Ala Trp Asp Pro Val Pro Ser Leu
   2570 2575 2580
   Ser Pro Ala Thr Thr Gly Ala Leu Ile Ser His Glu Lys Leu Leu
   2585 2590 2595
   Leu Gln Ile Asn Pro Glu Arg Glu Leu Gly Ser Met Ser Tyr Lys
   2600 2605 2610
   Leu Gly Gln Val Ser Ile His Ser Val Trp Leu Gly Asn Ser Ile
   2615 2620 2625
   Thr Pro Leu Arg Glu Glu Glu Trp Asp Glu Glu Glu Glu Glu Glu
   2630 2635 2640
   Ala Asp Ala Pro Ala Pro Ser Ser Pro Pro Thr Ser Pro Val Asn
   2645 2650 2655
   Ser Arg Lys His Arg Ala Gly Val Asp Ile His Ser Cys Ser Gln
   2660 2665 2670
   Phe Leu Leu Glu Leu Tyr Ser Arg Trp Ile Leu Pro Ser Ser Ser
   2675 2680 2685
   Ala Arg Arg Thr Pro Ala Ile Leu Ile Ser Glu Val Val Arg Ser
   2690 2695 2700
   Leu Leu Val Val Ser Asp Leu Phe Thr Glu Arg Asn Gln Phe Glu
   2705 2710 2715
   Leu Met Tyr Val Thr Leu Thr Glu Leu Arg Arg Val His Pro Ser
   2720 2725 2730
   Glu Asp Glu Ile Leu Ala Gln Tyr Leu Val Pro Ala Thr Cys Lys
   2735 2740 2745
   Ala Ala Ala Val Leu Gly Met Asp Lys Ala Val Ala Glu Pro Val
   2750 2755 2760
   Ser Arg Leu Leu Glu Ser Thr Leu Arg Ser Ser His Leu Pro Ser
   2765 2770 2775
   Arg Val Gly Ala Leu His Gly Val Leu Tyr Val Leu Glu Cys Asp
   2780 2785 2790
   Leu Leu Asp Asp Thr Ala Lys Gln Leu Ile Pro Val Ile Ser Asp
   2795 2800 2805
   Tyr Leu Leu Ser Asn Leu Lys Gly Ile Ala His Cys Val Asn Ile
   2810 2815 2820
   His Ser Gln Gln His Val Leu Val Met Cys Ala Thr Ala Phe Tyr
   2825 2830 2835
   Leu Ile Glu Asn Tyr Pro Leu Asp Val Gly Pro Glu Phe Ser Ala
   2840 2845 2850
   Ser Ile Ile Gln Met Cys Gly Val Met Leu Ser Gly Ser Glu Glu
   2855 2860 2865
   Ser Thr Pro Ser Ile Ile Tyr His Cys Ala Leu Arg Gly Leu Glu
   2870 2875 2880
   Arg Leu Leu Leu Ser Glu Gln Leu Ser Arg Leu Asp Ala Glu Ser
   2885 2890 2895
   Leu Val  Lys Leu Ser Val Asp  Arg Val Asn Val His  Ser Pro His
       2900                 2905                 2910
   Arg Ala  Met Ala Ala Leu Gly  Leu Met Leu Thr Cys  Met Tyr Thr
       2915                 2920                 2925
   Gly Lys  Glu Lys Val Ser Pro  Gly Arg Thr Ser Asp  Pro Asn Pro
       2930                 2935                 2940
   Ala Ala  Pro Asp Ser Glu Ser  Val Ile Val Ala Met  Glu Arg Val
       2945                 2950                 2955
   Ser Val  Leu Phe Asp Arg Ile  Arg Lys Gly Phe Pro  Cys Glu Ala
       2960                 2965                 2970
   Arg Val  Val Ala Arg Ile Leu  Pro Gln Phe Leu Asp  Asp Phe Phe
       2975                 2980                 2985
   Pro Pro  Gln Asp Ile Met Asn  Lys Val Ile Gly Glu  Phe Leu Ser
       2990                 2995                 3000
   Asn Gln  Gln Pro Tyr Pro Gln  Phe Met Ala Thr Val  Val Tyr Lys
       3005                 3010                 3015
   Val Phe  Gln Thr Leu His Ser  Thr Gly Gln Ser Ser  Met Val Arg
       3020                 3025                 3030
   Asp Trp  Val Met Leu Ser Leu  Ser Asn Phe Thr Gln  Arg Ala Pro
       3035                 3040                 3045
   Val Ala  Met Ala Thr Trp Ser  Leu Ser Cys Phe Phe  Val Ser Ala
       3050                 3055                 3060
   Ser Thr  Ser Pro Trp Val Ala  Ala Ile Leu Pro His  Val Ile Ser
       3065                 3070                 3075
   Arg Met  Gly Lys Leu Glu Gln  Val Asp Val Asn Leu  Phe Cys Leu
       3080                 3085                 3090
   Val Ala  Thr Asp Phe Tyr Arg  His Gln Ile Glu Glu  Glu Leu Asp
       3095                 3100                 3105
   Arg Arg  Ala Phe Gln Ser Val  Leu Glu Val Val Ala  Ala Pro Gly
       3110                 3115                 3120
   Ser Pro  Tyr His Arg Leu Leu  Thr Cys Leu Arg Asn  Val His Lys
       3125                 3130                 3135
   Val Thr  Thr Cys